# EUROPEAN PATENT APPLICATION

(11) **EP 4 696 367 A1**
(43) Date of publication of application: **18.02.2026**
(21) Application number: 25192305.8
(22) Date of filing: 29.07.2025
(51) Int. Cl.: A61N 1/365, A61N 1/372, A61B 5/01, A61N 1/362, A61N 1/375, A61N 1/39

(54) **SYSTEM FOR TEMPERATURE BASED DIAGNOSIS AND TREATMENT**

(30) Priority: 13.08.2024 US 202463682738 P; 25.07.2025 US 202519281153
(71) Applicant: Pacesetter, Inc., Sylmar, CA 91342 (US)
(72) Inventor: Pei, Xing, Thousand Oaks, CA (US); Wang, Hanbiao, Valley Glen, CA (US); Gill, Jong, Valencia, CA (US)
(74) Representative: Barker Brettell Sweden AB

(57) **Abstract**

A system (300) for temperature-based diagnosis and treatment is provided. The system (300) includes an IMD (100, 302) comprising a temperature sensor (253, 324) configured to collect treatment temperature data at a first rate when the IMD (100, 302) operates in a first mode and collect diagnostic temperature data at a second rate that is less than the first rate when the IMD (100, 302) operates in a second mode. The system (300) also includes one or more processors (306, 340) configured to analyze the treatment temperature data when the IMD (100, 302) is in the first mode and deliver a treatment of the IMD (100, 302) based on the treatment temperature data, and analyze the diagnostic temperature data when the IMD (100, 302) is in the second mode and communicate an alert based on the diagnostic temperature data.

## Description

### TECHNICAL FIELD

Embodiments of the present disclosure relate to systems for using patient temperature to provide diagnostics and modify treatments provided by implantable medical devices (IMDs).

### BACKGROUND

Cardiac implantable pacemakers and implantable cardioverter defibrillators (ICDs), as well as cardiac monitoring devices, are important devices for patients with heart conditions, because the devices serve to maintain the quality of life and substantially reduce mortality. These devices also provide monitoring functions for the cardiac and related physiological and pathophysiological signals.

Patients with an implantable pacemaker or ICD, cardiac monitoring devices, or the like may contract other diseases such as infections that can cause a fever. In addition, body temperature may also increase because of heat exposure or exercise. Given the commonality of infectious diseases and the extreme heat, it is not rare for the patient to experience elevated body temperatures from time to time.

Regardless of the underlying causes, when the body temperature increases above the normal temperature, a healthy heart increases the heart rate to boost the skin blood flow for faster heat-dissipation, as well as address other condition with cardiac output. A healthy heart can increase in heart rate more than 7 bpm for each 1 °C body temperature increase. However, patients with heart diseases and patients with compromised response may not have the ability to sufficiently increase their heart rate for compensation due to heart diseases when the elevated body temperature requires. The blunted heart rate response to the body temperature increase can result in degradation of the patient's quality of life, or even impact morbidity and mortality.

Heart diseases are often chronic, and gradually worsen over the years. The comprehensive patient health monitoring enables physicians to manage the heart diseases more effectively, and thus greatly improves the patient's quality of life and reduces costly hospitalization. The body temperature measurement is one of a set of holistic heart disease monitoring metrics, including heart rate, EGMs, thoracic impedance measurement, activity sensor data, and blood pressure measurement, glucose level, etc. Together, they can effectively detect the adverse conditions such as fevers due to infections, hyperthermia due to extreme heat exposure, and hypothermia due to worsening congestive heart failure etc.

### SUMMARY

The present invention is defined in the independent claim. Further embodiments of the invention are defined in the dependent claims.

In accordance with embodiments herein, a system for managing temperature-based diagnosis and treatment based on temperature data is provided. The system includes and implantable medical device (IMD) comprising a temperature sensor configured to collect treatment temperature data at a first rate when the IMD operates in a first mode and collect diagnostic temperature data at a second rate when the IMD operates in a second mode, wherein the first rate can be greater than the second rate. The system also includes one or more processors configured to analyze the treatment temperature data when the IMD is in the first mode and deliver a treatment of the IMD based on the treatment temperature data. The one or more processors can also be configured to analyze the diagnostic temperature data when the IMD is in the second mode and communicate an alert based on the diagnostic temperature data.

Optionally the system can also include a remote control in communication with the IMD, the remote control including one or more processors configured to receive the alert communicated by the IMD.

Optionally, the one or more processors of the remote control can be further configured to display the alert on a display along with the diagnostic temperature data.

Optionally, the remote control includes settings related to the treatment of the IMD, the diagnostic temperature data, and the treatment temperature data.

Optionally, the one or more processors of the remote control can be configured to switch the IMD from the first mode or second mode to a third mode where no temperature data is collected.

Optionally, the system can also include a physiological sensor configured to obtain physiological data. In the second mode the one or more processors can be further configured to identify a trend related to at least one of the diagnostic temperature data and the physiological data, identify a change in health status of a patient based on the trend, and communicate the alert in response to identifying the change in the health status.

Optionally, the physiological sensor can be at least one of a monitoring sensor, an activity sensor, or an impedance sensor. In another aspect, the IMD can include the physiological sensor.

Optionally, the trend can be at least one of a heart contraction characteristic trend, heart rate trend, QRS characteristic trend, capture threshold trend, lung impedance trend, tachycardiac arrythmia trend, or respiratory frequency trend.

Optionally, the one or more processors are configured to analyze the diagnostic temperature data by determining whether a change in a temperature threshold has been exceeded. The one or more processors are configured to communicate the alert based on the change in temperature threshold being exceeded.

Optionally, the one or more processors are configured to analyze the diagnostic temperature data by determining whether the temperature threshold has been exceeded for a duration threshold, and the one or more processors are configured to, in response to the duration threshold being exceeded, communicate the alert.

Optionally, the one or more processors can be configured to deliver the treatment of the IMD in the first mode based on the treatment temperature data using a first treatment profile for a determined period of time, and after the determined period of time, deliver the treatment of the IMD based on the treatment temperature data using a second treatment profile.

Optionally, the one or more processors are configured to, in respond to communicating the alert, change the IMD from operating in the second mode to operating in the first mode to collect the temperature data at the first rate.

Optionally, the IMD comprises the one or more processors.

Optionally, the system further comprises a remote control in communication with the IMD. In such an embodiment, the remote control comprises the one or more processors.

### BRIEF DESCRIPTION OF THE DRAWINGS

Figure 1A illustrates a schematic block diagram an implantable medical device (IMD) intended for subcutaneous implantation at a site near the heart.
Figure 1B illustrates an implantable medical device (IMD) provided for transvenous implantation in accordance with embodiments herein.
Figure 1C provides a sectional view of a patient's heart and shows a leadless implantable medical in accordance with embodiments herein.
Figure 1D illustrates a side view of the IMD according to an embodiment.
Figure 1E illustrates a graphical representation of an implantable medical system in accordance with embodiments herein.
Figure 2 illustrates a block diagram of an exemplary subcutaneous implantable medical device (S-IMD) that is configured to be implanted into the patient in accordance with embodiments herein.
Figure 3 illustrates a block diagram of an exemplary control system in accordance with embodiments herein.
Figure 4 illustrates a graph of daily exercise training, body temperature, and activity over time in accordance with embodiments herein.
Figure 5 illustrates a graph of pulmonary impedance and body temperature over time in accordance with embodiments herein.
Figure 6 illustrates a graph of body temperature fluctuations over time of AT/AF burden in accordance with embodiments herein.
Figure 7A-B illustrates a flow block diagram of the operation of an IMD in accordance with embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods and systems described herein may employ all or portions of structures or aspects of various embodiments discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, where indicated, the methods may be fully or partially implemented by one or more processors of one or more devices or systems. While the operations of some methods may be described as performed by the processor(s) of one device, additionally, some or all of such operations may be performed by the processor(s) of another device described herein.

### TERMS

The term "baseline temperature data," as used herein shall mean all data obtained by a temperature sensor of an IMD immediately after or in close proximity to after the implanting of an IMD. As an example, the baseline temperature data may be obtained within the first month after an IMD is implanted.

The term "treatment temperature data," as used herein shall mean all temperature data obtained by a temperature sensor of an IMD when the IMD is in a treatment mode.

The term "diagnostic temperature data," as used herein shall mean all temperature data obtained by a temperature sensor of an IMD when the IMD is in a diagnostic mode.

The term "treatment mode," as used herein shall mean a user setting in which treatment temperature data is obtained for the purpose of regulating, controlling, varying, changing, etc. the operation of the IMD to deliver treatment. In treatment mode a change in the body temperature of the patient can result in a change in the treatment delivered by the IMD.

The term "diagnostic mode," as used herein shall mean a user setting in which diagnostic temperature data is obtained for the purpose of diagnosing a disease, illness, condition, sickness, or the like. In diagnostic mode the diagnostic temperature data that is obtained can be logged, saved, communicated, or the like so that the diagnostic temperature data can be used to make diagnostic determinations.

The term "off mode," as used herein shall mean a user setting in which the temperature sensor of an IMD does not collect temperature data.

The term "obtain" or "obtaining", as used in connection with data, signals, information and the like, includes at least one of i) accessing memory of an external device or remote server where the data, signals, information, etc. are stored, ii) receiving the data, signals, information, etc. over a wireless communications link between the IMD and a local external device, and/or iii) receiving the data, signals, information, etc. at a remote server over a network connection. The obtaining operation, when from the perspective of an IMD, may include sensing new signals in real time, and/or accessing memory to read stored data, signals, information, etc. from memory within the IMD. The obtaining operation, when from the perspective of a local external device, includes receiving the data, signals, information, etc. at a transceiver of the local external device where the data, signals, information, etc. are transmitted from an IMD and/or a remote server. The obtaining operation may be from the perspective of a remote server, such as when receiving the data, signals, information, etc. at a network interface from a local external device and/or directly from an IMD. The remote server may also obtain the data, signals, information, etc. from local memory and/or from other memory, such as within a cloud storage environment and/or from the memory of a workstation or clinician external programmer.

The term "leadless" generally refers to an absence of electrically conductive leads that traverse vessels or other anatomy outside of the intra-cardiac space, while "intra-cardiac" means generally, entirely within the heart and associated vessels, such as the superior vena cava (SVC), inferior vena cava (IVC), coronary sinus (CS), coronary veins (CV), pulmonary arteries, and the like.

The term "real-time" refers to a time frame contemporaneous with normal or abnormal episode occurrences. For example, a real-time process or operation would occur during or immediately after (e.g., within seconds after, including within 3 seconds or 2 seconds after) a cardiac event, a series of cardiac events, an arrhythmia episode, and the like. For example, the term "real-time" may refer to a time period substantially contemporaneous with an event of interest. The term "real-time," when used in connection with collecting and/or processing data utilizing an IMD, shall refer to processing operations performed substantially contemporaneous with a physiologic event of interest experienced by a patient. By way of example, in accordance with embodiments herein, cardiac activity signals are analyzed in real-time (e.g., during a cardiac event or within a few seconds after the cardiac event).

### Overview

Embodiments may be implemented in connection with one or more implantable medical devices (IMDs). Non-limiting examples of IMDs include one or more therapy devices, and/or alternative implantable medical devices. For example, the IMD may represent a cardiac monitoring device, pacemaker, cardioverter, cardiac rhythm management device, defibrillator and the like. Additionally or alternatively, the IMD may include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

Additionally or alternatively, the IMD may be a subcutaneous IMD that includes one or more structural and/or functional aspects of the device(s) described in U.S. Patent 10,765,860, titled "Subcutaneous Implantation Medical Device With Multiple Parasternal-Anterior Electrodes" and filed May 7, 2018; U.S. Patent 10,722,704, titled "Implantable Medical Systems And Methods Including Pulse Generators And Leads" filed May 7, 2018; US Patent 11,045,643, titled "Single Site Implantation Methods For Medical Devices Having Multiple Leads", filed May 7, 2018. Further, one or more combinations of IMDs may be utilized from the above mentioned patents and applications in accordance with embodiments herein.

In accordance with embodiments herein methods and systems are described that are IMDs that provide a programmable feature that regulates physiological treatment of a patient, such as regulating a pacing rate, delay, or the like based on the body temperature of the patient to mimic the natural cardiac response to changes in body temperature. Modern medical device remote monitoring technology can correlate multiple types of measurement trend data, alert the physician of such adverse conditions in real-time, and enable the physicians to provide timely intervention or therapy. In addition, the trending diagnostics can include temperature and/or temperature change as one additional factor that can affect the heart conditions and the association of the disease status change, as well as provide clinical alerts to facilitate the patient management for necessary earlier intervention and provide more safety to the patient.

The system provided herein can have a temperature sensor on a lead, or alternatively in a different location of the patient, to measure the core temperature inside the heart and/or body temperature. In one example, the IMD can be a pacer device system where a temperature sensor is placed in the pacemaker device close to the body surface. The peripheral temperature not only follows the core heart temperature change that is driven by the body internal metabolism and disease condition, but also can effectively detect the body exposure to extreme heat.

The system does not use the temperature as an activity sensor or determine an activity level for controlling the heart rate. Instead, the system uses temperature (which can be higher or lower than the patient base temperature) and temperature changes as a compensatory heart rate regulation to address the disease status change for cardiac output compensation. The system combines various long term and circadian variations of the temperature, along with other long-term trends, to provide a clinician with additional data for diagnostic analysis for when body temperature changes (e.g., excursion) occurs. Temperature information and data related to temperature changes of the patient can be utilized to provide a medical alert mechanism for clinicians. In one example, the temperature information and data can be used to reduce or resolve potential false positives related to medical alerts provided by the system. As an example, a serious illness like hypothermia or sepsis can require immediate attention where an incorrect temperature reading of another temperature device can result in a false alert. Alternatively, a patient may have another underlying factor contributing to the lower temperature like advanced age, medication use, or hypothyroidism that is not the result of hypothermia or sepsis, resulting in the false alert.

While the system can be used to determine false alerts related to hypothermia, the system can also be utilized to detect hypothermia more readily and accurately. Closely regulated temperature is imperative for normal and efficient functioning of organ systems. Drastic and irreparable changes in organ structure and function can occur when body temperature falls below 32.2°C (90°F) or rises above 41.1°C (106°F). Lesser changes result in confusion, delirium, seizures, or cardiorespiratory embarrassment, depending on the physical status and age of the host. However, one could point to remarkable recovery of patients with temperature documented below 18°C (65°F) or above 44.5°C (112°F).

Hypothermia can be due to infection and bacteremia, ethanol or drug ingestion, exposure, a central nervous system event, cachexia from malignancy or malnutrition, gastrointestinal bleeding, or endocrine deficiencies such as panhypopituitarism, myxedema, Addison's disease, uremia, and hypoglycemia. In some patients with these diseases, the febrile state might not be recognized because they will raise their temperature to less than 37.2°C (99°F). The early stage of hypothermia (35° to 32.8°C; 95° to 91°F) is marked by an attempt to react against chilling including shivering, increased blood pressure and pulse, vasoconstriction, and diuresis. An intermediate stage (32.2° to 24°C; 90° to 75°F) is characterized by decrease in metabolism; drop in pulse, blood pressure, and respiration; muscular rigidity; a fine tremor; and respiratory and metabolic acidosis. At a third stage, when all attempts at compensation by the temperature regulatory center fail, the body loses heat like an inanimate object. Most patients with hypothermia exhibit tachycardia, tachypnea, hypotension, leukocytosis, acidosis, increased pulmonary wedge pressure, and right atrial pressure; patients with hypothermia caused by infection with bacteremia have much lower systemic vascular resistance and higher cardiac index than nonbacteremic patients with hypothermia. By providing accurate temperature sensing and trending, the system can improve diagnosis and related treatment for hypothermia.

The system can also detect other physiological conditions because of body temperature changes. For example, although most increases in body temperature seen in the clinic are fevers, changing lifestyles, old age, and medical treatments can be responsible for an increasing number of patients with hyperthermia. Fever persisting for more than 2 weeks, especially when it is accompanied by malaise, anorexia, and weight loss, requires thorough consideration and investigation. Often the persistent fever results in a patient to encounter a hospital setting with relatively invasive and expensive tests. Prerequisite to the development of an effective diagnostic plan is a detailed history and careful and complete physical examination with frequent, often daily, reassessment of selected parameters. A methodical and sequential laboratory evaluation is essential. The system provided herein can provide additional monitoring and temperature data, reducing the need for hospital visits, and additional expenses.

In yet another example, temperature changes can include decreases as a result of a patient not being active and the heart is not able to pump out the blood effectively). The system can additionally monitor, diagnose, and treat physiologic conditions as a result of detecting changes in temperature over time.

### Implantable Medical Devices and Control Systems

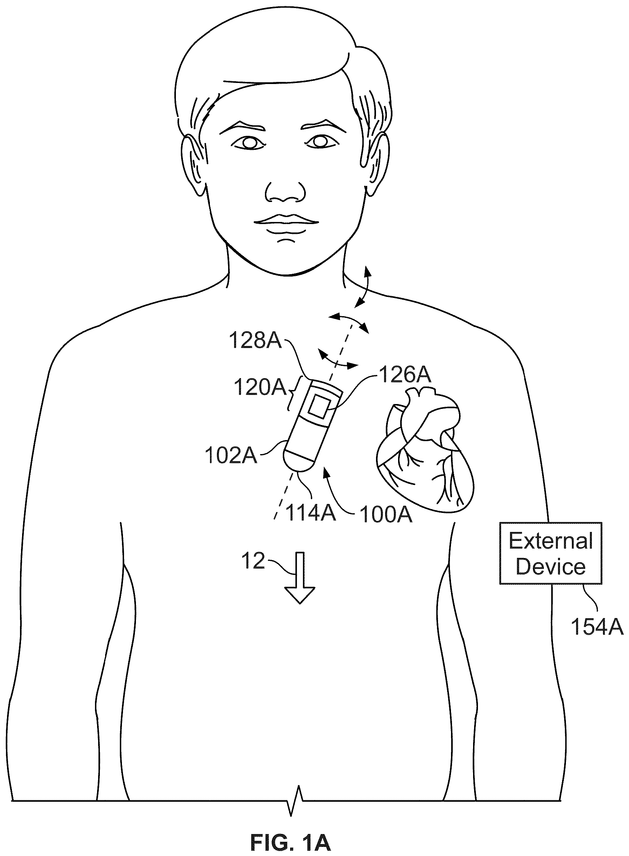

Figure 1A illustrates an implantable medical device (IMD) 100A intended for subcutaneous implantation at a site near the heart. In one example, the IMD and related sensors, devices, systems, etc. as described herein is at least one of the IMDs, sensors, devices, systems, etc. illustrated and described in U.S. Patent 12,161,503, filed February 8, 2022, entitled METHOD AND SYSTEM FOR MONITORING HEART FUNCTION BASED ON HEART SOUND CENTER OF MASS. In another example, the IMD and related sensors, devices, systems, etc. as described herein is at least one of the IMDs, sensors, devices, systems, etc. illustrated and described in U.S. Patent Publication 2022/0361818, filed February 8, 2022, entitled METHOD AND SYSTEM FOR MONITORING HEART FUNCTION BASED ON HEART SOUND CENTER OF MASS.

The IMD 100A includes a pair of spaced-apart sense electrodes 114A, 126A positioned with respect to housing 102A. The sense electrodes 114A, 126A provide for detection of far field electrogram signals. Numerous configurations of electrode arrangements are possible. For example, the electrode 114A may be located on a distal end of the IMD 100A, while the electrode 126A is located on a proximal side of the IMD 100A. Additionally or alternatively, electrodes 126A may be located on opposite sides of the IMD 100A, opposite ends or elsewhere. The distal electrode 114A may be formed as part of the housing 102A, for example, by coating all but a portion of the housing with a nonconductive material such that the uncoated portion forms the electrode 114A. In this case, the electrode 126A may be electrically isolated from the housing 102A electrode by placing it on a component separate from the housing 102A, such as the header 120A. Optionally, the header 120A may be formed as an integral portion of the housing 102A. The header 120A includes an antenna 128A and the electrode 126A. The antenna 128A is configured to wirelessly communicate with an external device 154A in accordance with one or more predetermined wireless protocols (e.g., Bluetooth, Bluetooth low energy, Wi-Fi, etc.). In one example, the external device 154A is a proximate external device located proximate to or adjacent to a patient. An external device located in the same room as the patient is a proximate external device. Alternatively, the external device can be a remote external device where the external device is located a long distance from the such as at least one-hundred meters, in a different room, in a different building, at least five miles away, at least ten miles away, at least one-hundred miles away, etc.

The housing 102A includes various other components such as: sense electronics for receiving signals from the electrodes, a microprocessor for analyzing the far field cardiac activity (CA) signals, including assessing the presence of R-waves in cardiac beats occurring while the IMD is in different IMD locations relative to gravitational force, a loop memory for temporary storage of CA data, a device memory for long-term storage of CA data, sensors for detecting patient activity, including an accelerometer for detecting acceleration signatures indicative of heart sound, and a battery for powering components.

In at least some embodiments, the IMD 100A is configured to be placed subcutaneously utilizing a minimally invasive approach. Subcutaneous electrodes are provided on the housing 102A to simplify the implant procedure and eliminate a need for a transvenous lead system. The sensing electrodes may be located on opposite sides of the device and designed to provide robust episode detection through consistent contact at a sensor-tissue interface. The IMD 100A may be configured to be activated by the patient or automatically activated, in connection with recording subcutaneous ECG signals.

The IMD 100A senses far field, subcutaneous CA signals, processes the CA signals to detect arrhythmias and if an arrhythmia is detected, automatically records the CA signals in memory for subsequent transmission to an external device 154A.

The IMD 100A is implanted in a position and orientation such that, when the patient stands, the IMD 100A is located at a reference position and orientation with respect to a global coordinate system that is defined relative to a gravitational direction 12. For example, the gravitational direction 12 is along the Z-axis while the X-axis is between the left and right arms.

As explained herein, the IMD 100A includes electrodes that collect CA signals in connection with multiple cardiac beats and in connection with different IMD locations (e.g., different positions and/or different orientations). The IMD 100 also includes one or more sensors to collect acceleration signatures that are indicative of heart sounds produced at different points in a cardiac cycle.

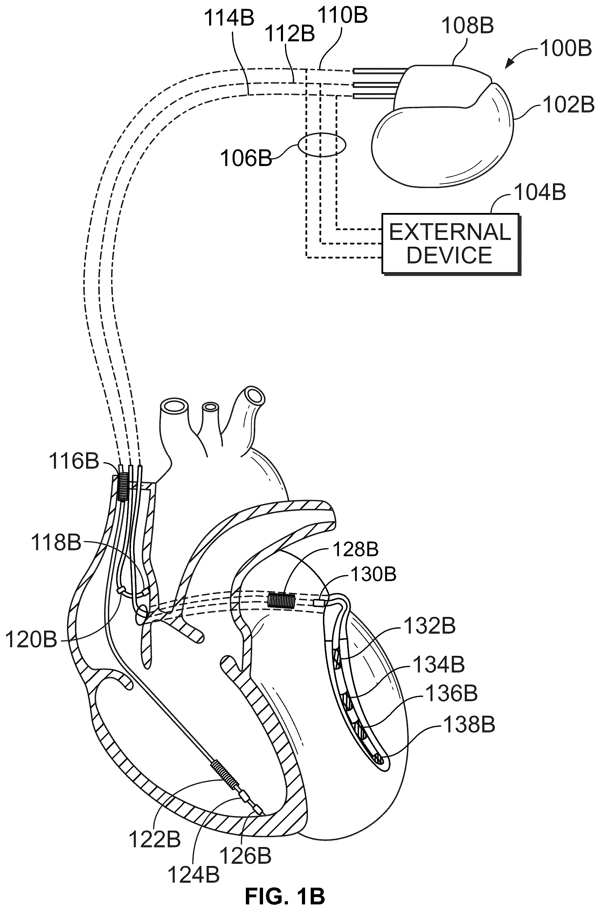

Figure 1B illustrates an alternative IMD 100B that may apply treatment, such as a shock. The IMD 100B may be a dual-chamber stimulation device capable of treating both fast and slow arrhythmias with stimulation therapy, including cardioversion, defibrillation, anti-tachycardia pacing and pacing stimulation, as well as capable of detecting heart failure, evaluating its severity, tracking the progression thereof, and controlling the delivery of therapy and warnings in response thereto. The IMD 100B may be controlled to sense atrial and ventricular waveforms of interest, discriminate between two or more ventricular waveforms of interest, deliver stimulus pulses or shocks, and inhibit application of a stimulation pulse to a heart based on the discrimination between the waveforms of interest and the like. Exemplary structures for the monitoring device 100B are discussed and illustrated in the drawings herewith.

The IMD 100B includes a housing 102B that is joined to a header assembly 108B that holds receptacle connectors connected to a right ventricular lead 110B, a right atrial lead 112B, and a coronary sinus lead 114B, respectively. The leads 112B, 114B and 110B measure cardiac signals of the heart. The right atrial lead 112B includes an atrial tip electrode 118B and an atrial ring electrode 120B. The coronary sinus lead 114B includes a left atrial ring electrode 128B, a left atrial coil electrode 130B and one or more left ventricular electrodes 132B-138B (e.g., also referred to as P1, M1, M2 and D1) to form a multi-pole LV electrode combination. The right ventricular lead 110B includes an RV tip electrode 126B, an RV ring electrode 124B, an RV coil electrode 122B, and an SVC coil electrode 116B. The leads 112B, 114B and 110B detect IEGM signals that are processed and analyzed as described herein. The leads 112B, 114B and 110B also delivery therapies as described herein.

During implantation, an external device 104B is connected to one or more of the leads 112B, 114B and 110B through temporary inputs 106B. The inputs 106B of the external device 104B receive IEGM signals from the leads 112B, 114B and 110B during implantation and display the IEGM signals to the physician on a display. Hence, the external device 104B receives the IEGM cardiac signals through telemetry circuit inputs. The physician or another user controls operation of the external device 104B through a user interface.

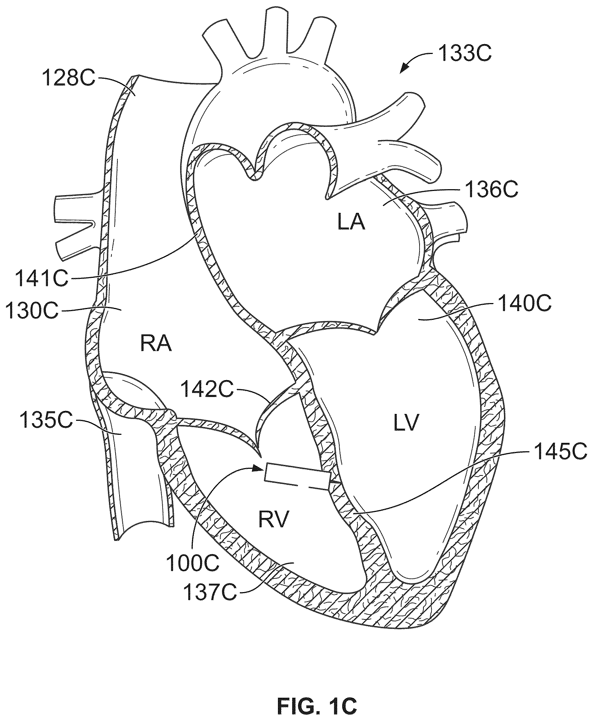

Figure 1C provides an alternative embodiment of an IMD 100C. Figure 1C illustrates a sectional view of a patient's heart 133C and shows a leadless IMD 100C. The IMD 100C has been placed through the superior vena cava 128C into the right atrium 130C of the heart 133C. Figure 1C also shows the inferior vena cava 135C, the left atrium 136C, the right ventricle 137C, the left ventricle 140C, the atrial septum 141C that divides the two atria 130C, 136C, and the tricuspid valve 142C between the right atrium 130C and the right ventricle 137C.

The IMD 100C is formed in accordance with an embodiment. The IMD 100C may represent a pacemaker, a cardiac resynchronization therapy (CRT) device, a cardioverter, a cardiac rhythm management (CRM) device, a defibrillator, or the like. The IMD 100C comprises a housing 102C configured to be implanted entirely within a single local chamber of the heart 133C, such as entirely and solely within the right atrium 130C, left atrium 136C, the right ventricle 137C or the left ventricle 140C, for example. Optionally, the IMD 100C may be implanted outside of the chambers of the heart but located in a vessel proximate to, or attached to an exterior wall of, the heart proximate to the RA, LA, RV or LV.

The chamber in which the IMD 100C is implanted in (or closest proximally to) is referred to as the "local" chamber. The local chamber includes a local chamber wall that is physiologically responsive to local activation events originating in the local chamber. The local chamber is at least partially surrounded by local wall tissue that forms, contains, or constitutes at least part of a conduction network for the associated chamber.

As shown in Figure 1C, the local chamber in which the IMD 100C is implanted is the right ventricle 137C. For example, the IMD 100C is mounted or fixated to the tissue wall of the right ventricle 137C along the septum 145C that divides the right ventricle 137C from the left ventricle 140C. The septum 145C wall tissue in the right ventricle 137C may behave physiologically differently than the non-septum ventricular wall tissue. Optionally, the IMD 100C may be implanted in other regions of the RV, in other chambers of the heart, in vessels along an exterior of a local chamber or implanted in the exterior wall of the heart proximate to a local chamber (e.g., through the epicardium and into the myocardium). Figure 1C shows the IMD 100C in the septal wall of the RV, but optionally, the IMD 100C may be implanted at a higher location proximate to the HIS bundle in the RV. Optionally, the IMD 100C may be implanted in the RA or elsewhere. Alternatively, multiple IMDs may be implanted into the patient's heart 133C within different chambers or different segments of the same chamber.

The leadless IMD 100C may sense various intrinsic events and delivery corresponding therapies depending upon whether a subsequent intrinsic event is detected within a certain time period. For example, the IMD may utilize one or more atrial-ventricular (AV) delays to manage ventricular pacing in the event an intrinsic ventricular event does not occur within a programmed time period following a preceding intrinsic (or paced) atrial event. Similarly, the IMD may utilize one or more ventricular-ventricular (VV) delays to manage synchronization between right and left side ventricular activity. For example, a leadless IMD in the RV may deliver a paced event when an intrinsic right side ventricular event does not occur within a programmed time period following a preceding intrinsic (or paced) ventricular event in the left ventricular chamber, or vice versa. As another example, the IMD may utilize one or more atrial-HIS (AH) delays to manage HIS bundle pacing in the event an intrinsic event does not occur at the HIS bundle within a programmed time period following a preceding intrinsic (or paced) atrial event. As another example, when the IMD is implanted in the atrium, the IMD may utilize one or more post ventricular atrial refractory period (PVARP) blanking intervals to manage blanking for a sensing circuit following a preceding intrinsic ventricular event.

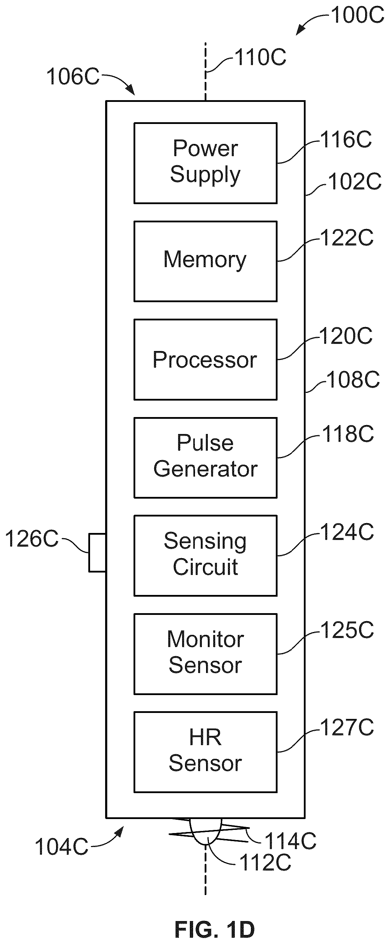

Figure 1D illustrates a side view of the IMD 100C according to an embodiment. The illustrated IMD 100C includes a schematic representation of some internal components of the IMD 100C. The housing 102C of the IMD 100C includes a first mounting end 104C, an opposite second end 106C, and an intermediate shell 108C extending between the first end 104C and the second end 106C. The shell 108C is elongated and tubular in shape and extends along a longitudinal axis 110C. The mounting end 104C mounts to tissue of an intra-cardiac wall within a chamber of the heart.

The mounting end 104C includes an electrode 112C securely attached thereto and projecting outward from the mounting end 104C. The shell 108C includes one or more electrodes 126C provided therein remote from the electrode 112C. The electrodes 112C and 126C cooperate to define a sensing vector and to sense local CA signals. The electrodes 112C and 126C are further configured to deliver stimulation energy to tissue of interest. As used herein, "tissue of interest" refers to intra-cardiac tissue that the IMD 100C is configured to monitor and provide stimulation energy. In the illustrated embodiment, the IMD 100C is configured to be affixed directly to the tissue of interest, as described below. The electrode 112C may be a cathode electrode that is actively fixated to the myocardium, while the electrode 126C is an anode electrode. The stimulation energy may be in the form of low-energy pacing pulses, higher-energy shocking pulses, or the like.

When the mounting end 104C is mounted to the intra-cardiac tissue, the electrode 112C is securely affixed to and engages the tissue of interest in order to deliver the stimulation energy directly thereto. In addition to delivering stimulation energy, in an alternative embodiment the electrode 112C may be used to sense electrical activity from the tissue of interest. The electrode 112C may be formed as a single conductive bulb or, alternatively, as a cone, a single wire, or the like. Optionally, the electrode 112C is not covered with insulation material and the conductive material is exposed in order to facilitate a good electrical connection to the local wall tissue. Alternatively, at least a portion of the electrode 112C is covered with insulation to prevent electrical conduction to tissue that engages the insulation.

The mounting end 104C includes a fixation element to secure the IMD in or proximate to a local chamber of the heart. For example, the fixation element may be a fixation screw 114C securely attached thereto and projecting outward from the mounting end 104C. The fixation screw 114C is configured to extend into the tissue of interest to anchor the IMD 100C to the intra-cardiac tissue. The fixation screw 114C is configured to be screwed into the tissue to firmly adhere the IMD 100C thereto by pressing the mounting end 104C against the tissue and rotating the IMD 100C in a first, coupling direction. The fixation screw 114C may be extracted from the tissue by rotating the IMD 100C in an opposite, uncoupling direction in conjunction with a slight tugging force directed away from the myocardial wall. The fixation screw 114C may be shaped as a helical corkscrew that defines a center channel. For example, the fixation screw 114C may surround the electrode 112C such that the electrode 112C is within the center channel. In an alternative embodiment, the fixation screw 114C is part of the electrode 112C. For example, the electrode 112C may have helical threads on an outer surface of the electrode 112C, such that the electrode 112C forms the fixation screw 114C.

Additionally or alternatively, the fixation element may include one or more loops, tabs and the like that are configured to retain the IMD in a vessel, septal wall or other tissue proximate to the local chamber of the heart. For example, the IMD and/or fixation element may be formed as described in one or more of U.S. Patent Publication 2019/0099087, published April 4, 2019, and titled "Wireless Sensor for Measuring Pressure"; U.S. Patent 9,993,167, issuing June 12, 2018 and titled "Apparatus and Method for Sensor Deployment and Fixation"; U.S. Patent Publication 2016/0007924, published January 14, 2016, and titled "Implantable Pressure Transducer System Optimized to Correct Environmental Factors".

The housing 102C retains a power supply 116C and various electronic components that receive electrical current from the power supply 116C. The electronic components provide the functionality of the IMD 100C, such as controlling the stimulation energy delivered to the electrode 112C and sensing the depolarization along the tissue of interest in response to a pacing pulse or to an intrinsic heartbeat. The power supply 116C stores charge for gradual disbursal to the electronic components as needed. The power supply 116C may be a battery. The power supply 116C has a fixed amount of charge at full capacity. The power supply 116C may be rechargeable in some embodiments and may not be rechargeable in other embodiments. The power supply 116C is fully retained within and surrounded by the housing 102C.

The electronic components include a pulse generator 118C, a processor 120C, a memory 122C, a sensing circuit 124C, a monitoring sensor/system 125C, and heart rate (HR) sensor 127C. In one example the monitoring sensor can include a temperature sensor, a physiological sensor, or the like. The illustration is intended as an overview of the electronic components only, and the electronic components according to an embodiment of the IMD 100C. The pulse generator 118C provides stimulation energy to the electrode 112C which is delivered to the tissue of interest that the electrode 112C engages. The pulse generator 118C includes circuitry to control the output of stimulation energy directed to the electrode 112C. For example, the pulse generator 118C produces lower energy pulses for pacing and higher energy pulses for shocking.

The processor 120C is a controller that controls the flow of charge between the power supply 116C, the electronic components (such as the pulse generator 118C, monitoring sensor 125C and the sensing circuit 124C), and the electrodes (such as electrode 112C). For example, the processor 120C controls the timing and intensity or magnitude of the stimulation pulses. If multiple electrodes are used to deliver stimulation energy to the intra-cardiac tissue, the processor 120C may synchronize the delivery of the pulses. The processor 120C is communicatively coupled to the pulse generator 118C, the sensing circuit 124C, the memory 122C, and the power supply 116C. The processor 120C also functions based on instructions stored locally in the memory 122C. The memory 122C is a non-transitory tangible computer readable storage medium. The memory 122C stores programmable and executable instructions for the processor 120C. The processor 120C is responsive to the programmable instructions to control operation of the IMD 100C as described herein. The memory 122C may also store data. Some of the data may be stored prior to completing assembly of the IMD 100C, while other data may be stored during use of the implanted IMD 100C. For example, the memory 122C may be used to store data on intrinsic electrical activity within the heart as monitored by the sensing circuit 124C, data on the number, time, and/or magnitude of pacing pulses generated by the pulse generator 118C, or the like. The memory 122C is further configured to store temperature signals, temperature data, temperature maximums, temperature minimums, temperature averages, temperature medians, and the like.

The sensing circuit 124C is configured to monitor intrinsic electrical CA signals within the heart. The sensing circuit 124C is communicatively coupled to one or more sensing electrodes 112C, 126C located on or extending from the housing 102C. The sensing electrodes 126C are shown located along one or more sides of the shell 108C but may additionally or alternatively be located along the second end 106C of the housing 102C. The sensing electrode 126C senses electrical activity, such as physiologic and pathologic behavior and events, and provides sensed signals to the sensing circuit 124C in response. In an alternative embodiment, the pulsing electrode 112C doubles as a sensing electrode, such that the pulsing electrode 112C is used to deliver stimulation pulses and, in-between pulses, monitors the electrical activity within the tissue of interest for the sensing circuit 124C.

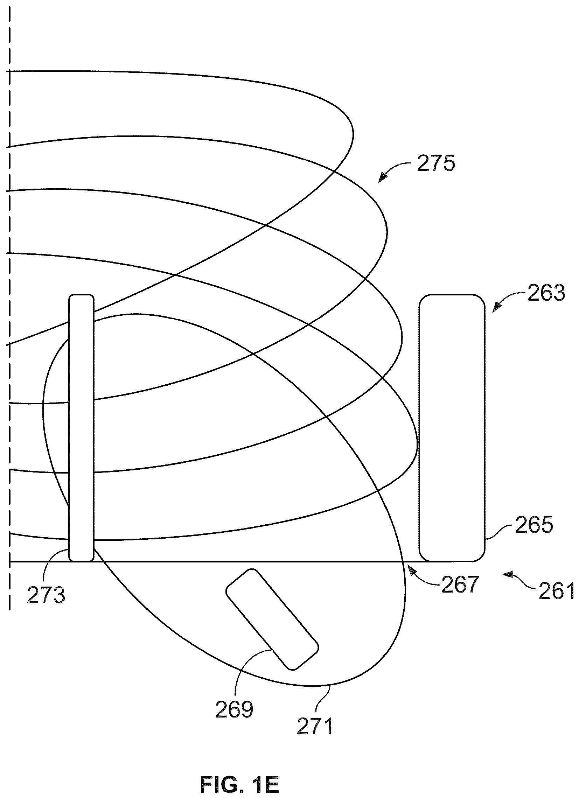

Figure 1E illustrates a representation of yet another example of an implantable medical system 261. This implantable medical system 261 is configured to apply therapy in accordance with embodiments herein. Embodiments may be implemented in connection with one or more subcutaneous implantable medical devices (S-IMDs). Non-limiting examples of S-IMDs include one or more of subcutaneous implantable cardioverter defibrillators (S-ICD). For example, the S-IMD may include one or more structural and/or functional aspects of the device(s) described in U.S. Patent 10,722,704, titled "IMPLANTABLE MEDICAL SYSTEMS AND METHODS INCLUDING PULSE GENERATORS AND LEADS", filed May 07, 2018; U.S. Patent 10,765,860, titled "SUBCUTANEOUS IMPLANTATION MEDICAL DEVICE WITH MULTIPLE PARASTERNAL-ANTERIOR ELECTRODES", filed May 07, 2018.

The system 261 includes an S-IMD 263 that is configured to be implanted in a subcutaneous area exterior to the heart. The S-IMD 263 is positioned in a subcutaneous area or region, and more particularly in a mid-axillary position along a portion of the rib cage 275. Optionally, the system 261 may also include a leadless pacemaker 269 implanted within the heart, such as at an apex 271 of the right ventricle. Optionally, the leadless pacemaker 269 may be omitted entirely. The system 261 does not require insertion of a transvenous lead.

The pulse generator 265 may be implanted subcutaneously and at least a portion of the lead 267 may be implanted subcutaneously. In particular embodiments, the S-IMD 263 is an entirely or fully subcutaneous S-IMD. Optionally, the S-IMD 263 may be positioned in a different subcutaneous region.

The S-IMD 263 includes a pulse generator 265 and at least one lead 267 that is operably coupled to the pulse generator 265. The lead 267 includes at least one electrode segment 273 that is used for providing shocks for defibrillation. Optionally, the lead 267 may include one or more sensing electrodes. The pulse generator 265 includes a housing that forms or constitutes an electrode utilized to deliver shocks. The electrode associated with the housing of the pulse generator 265 is referred to as the "CAN" electrode.

In an alternative embodiment, the lead 267 may include one or more electrode segments, in which the electrode segments are spaced apart from one another having an electrical gap therebetween. The lead body may extend between the gap. One electrode segment may be positioned along an anterior of the chest, while another electrode segment may be positioned along a lateral and/or posterior region of the patient. The electrode segments may be portions of the same lead, or the electrode segments may be portions of different leads. The electrode segments may be positioned subcutaneously at a level that aligns with the heart of the patient for providing a sufficient amount of energy for defibrillation. The lead includes a lead body that extends from the mid-auxiliary position along an inter-costal area between ribs and oriented with the coil electrode(s) extending along the sternum (e.g., over the sternum or parasternally within one to three centimeters from the sternum). A proximal end the coil electrodes may be located proximate to the xiphoid process.

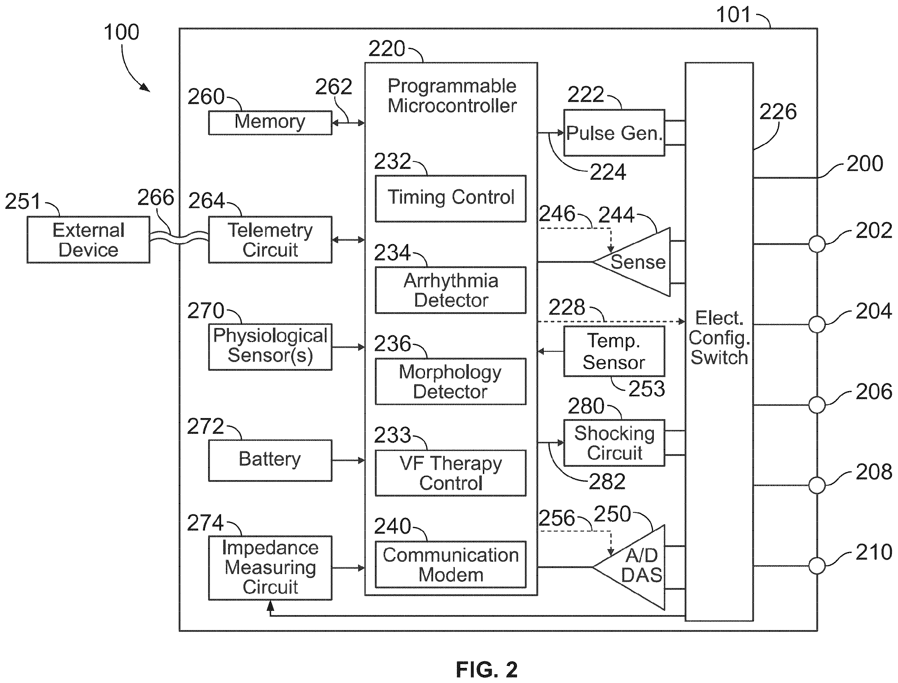

Figure 2 shows a block diagram of an exemplary S-IMD 100 that is configured to be implanted into the patient. The S-IMD 100 may treat both fast and slow arrhythmias with stimulation therapy, including cardioversion, pacing stimulation, an implantable cardioverter defibrillator, suspend tachycardia detection, tachyarrhythmia therapy, and/or the like.

The S-IMD 100 has a housing 101 to hold the electronic/computing components. The housing 101 (which is often referred to as the "can," "case," "encasing," or "case electrode") may be programmably selected to act as the return electrode for certain stimulus modes. The housing 101 further includes a connector (not shown) with a plurality of terminals 200-210. The terminals may be connected to electrodes that are located in various locations within and about the heart. The type and location of each electrode may vary. For example, the electrodes may include various combinations of ring, tip, coil, shocking electrodes, and the like.

The S-IMD 100 includes a programmable microcontroller 220 that controls various operations of the S-IMD 100, including cardiac monitoring and stimulation therapy. The microcontroller 220 includes a microprocessor (or equivalent control circuitry), one or more processors, RAM and/or ROM memory, logic and timing circuitry, state machine circuitry, and I/O circuitry. The S-IMD 100 further includes a ventricular pulse generator 222 that generates stimulation pulses for connecting the desired electrodes to the appropriate I/O circuits, thereby facilitating electrode programmability. The switch 226 is controlled by a control signal 228 from the microcontroller 220.

A pulse generator 222 is illustrated in Figure 2A. Optionally, the S-IMD 100 may include multiple pulse generators, similar to the pulse generator 222, where each pulse generator is coupled to one or more electrodes and controlled by the microcontroller 220 to deliver select stimulus pulse(s) to the corresponding one or more electrodes. The S-IMD 100 includes sensing circuit 244 selectively coupled to one or more electrodes that perform sensing operations, through the switch 226 to detect the presence of cardiac activity in the chamber of the heart 111. The output of the sensing circuit 244 is connected to the microcontroller 220 which, in turn, triggers, or inhibits the pulse generator 222 in response to the absence or presence of cardiac activity. The sensing circuit 244 receives a control signal 246 from the microcontroller 220 for purposes of controlling the gain, threshold, polarization charge removal circuitry (not shown), and the timing of any blocking circuitry (not shown) coupled to the inputs of the sensing circuit 244.

In the example of Figure 2A, the sensing circuit 244 is illustrated. Optionally, the S-IMD 100 may include multiple sensing circuits 244, where each sensing circuit is coupled to one or more electrodes and controlled by the microcontroller 220 to sense electrical activity detected at the corresponding one or more electrodes. The sensing circuit 224 may operate in a unipolar sensing configuration or a bipolar sensing configuration.

The S-IMD 100 further includes an analog-to-digital (A/D) data acquisition system (DAS) 250 coupled to one or more electrodes via the switch 226 to sample cardiac signals across any pair of desired electrodes. The A/D converter 250 is configured to acquire intracardiac electrogram signals, convert the raw analog data into digital data and store the digital data for later processing and/or telemetric transmission to an external device 251 (e.g., a programmer, local transceiver, or a diagnostic system analyzer). The A/D converter 250 is controlled by a control signal 256 from the microcontroller 220. In one example the external device 251 can be configured to be a remote control that can be used to control settings of the S-IMD 100, vary settings, provide programming, receive data and information from the S-IMD, or the like.

The switch 226 may be coupled to an LV lead having multiple LV electrodes, at least one of the LV electrodes configured to be located proximate to the LV site corresponding to the pacing site and to deliver the burst pacing therapy. The switch 226 may be further coupled to a second lead with at least one of a superior vena cava (SVC) coil electrode or an RV coil electrode, the shock vector including a CAN of the S-IMD and at least one of the SVC coil electrode or the RV coil electrode.

The microcontroller 220 is operably coupled to a memory 260 by a suitable data/address bus 262. The programmable operating parameters used by the microcontroller 220 are stored in the memory 260 and used to customize the operation of the S-IMD 100 to suit the needs of a particular patient. The operating parameters of the S-IMD 100 may be non-invasively programmed into the memory 260 through a telemetry circuit 264 in telemetric communication via communication link 266 (e.g., MICS, Bluetooth low energy, and/or the like) with the external device 251.

The S-IMD 100 can further include one or more physiological sensors 270 that are configured to obtain physiological data. As used herein, the physiological sensors 270 can monitor, detect, sensor, etc. any physiologic characteristic, except for temperature. In examples, the physiological sensor(s) 270 can include a monitoring sensor such as an ECG that obtains cardiac activity signals. In another example the monitoring sensor can be a blood pressure sensor. In another examples, the physiological sensor can be an activity sensor that is configured to measure the amount of activity of a patient. In one example the activity sensor is an accelerometer that measures the motion and posture of the patient. In yet another example the physiological sensor(s) can be an impedance sensor configured to monitor respiration via impedance.

Such sensors are commonly referred to as "rate-responsive" sensors because they are typically used to adjust pacing stimulation rates according to the state of the patient. However, the physiological sensor 270 may further be used to detect changes in cardiac output, changes in the physiological condition of the heart, or diurnal changes in activity (e.g., detecting sleep and wake states). Signals generated by the physiological sensors 270 are passed to the microcontroller 220 for analysis. While shown as being included within the S-IMD 100, the physiological sensor(s) 270 may be external to the S-IMD 100, yet still, be implanted within or carried by the patient. Examples of physiological sensors might include sensors that, for example, sense respiration rate, pH of blood, ventricular gradient, activity, position/posture, minute ventilation, and/or the like.

In addition to the physiological sensors 270, the S-IMD 100 includes a temperature sensor 253 that can be configured to obtain signals related to the body temperature of the patient. In example embodiments the temperature sensor 253 can be coupled to a lead, not coupled to a lead and part of a leadless device or system, coupled directly to the S-IMD, coupled remotely from the S-IMD, or the like. By obtaining temperature signals, changes in body temperature can be detected and analyzed to determine whether any changes in pacing, rate, etc. or if any adjustments, modifications, changes, or the like should be made to treatments provided by the S-IMD 100.

A battery 272 provides operating power to all of the components in the S-IMD 100. The battery 272 is capable of operating at low current drains for extended periods of time and is capable of providing a high-current pulses (for capacitor charging) when the patient requires a shock pulse (e.g., in excess of 2 A, at voltages above 2 V, for periods of 10 seconds or more). The battery 272 also desirably has a predictable discharge characteristic so that elective replacement time can be detected. As one example, the S-IMD 100 employs lithium/silver vanadium oxide batteries.

The S-IMD 100 further includes an impedance measuring circuit 274, which can be used for many things, including sensing respiration phase. The impedance measuring circuit 274 is coupled to the switch 226 so that any desired electrode and/or terminal may be used to measure impedance in connection with monitoring respiration phase. The S-IMD 100 is further equipped with a communication modem (modulator/demodulator) 240 to enable wireless communication with other devices, implanted devices and/or external devices. In one implementation, the communication modem 240 may use high frequency modulation of a signal transmitted between a pair of electrodes. As one example, the signals may be transmitted in a high frequency range of approximately 10-80 kHz, as such signals travel through the body tissue and fluids without stimulating the heart or being felt by the patient.

The microcontroller 220 further controls a shocking circuit 280 by way of a timing control 232. The shocking circuit 280 generates shocking pulses as controlled by the microcontroller 220. The shocking circuit 280 is controlled by the microcontroller 220 by a control signal 282.

Although not shown, the microcontroller 220 may further include other dedicated circuitry and/or firmware/software components that assist in monitoring various conditions of the patient's heart and managing pacing therapies. The microcontroller 220 further includes a timing control 232, an arrhythmia detector 234, a morphology detector 236 and multi-phase VF therapy controller 233. The timing control 232 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The timing control 232 controls a timing for delivering the phase I, II and III therapies in a coordinated manner.

The morphology detector 236 is configured to review and analyze one or more features of the morphology of CA signals. For example, in accordance with embodiments herein, the morphology detector 236 may analyze the morphology of detected R-waves, where such morphology is then utilized to determine whether to include or exclude one or more beats from further analysis. For example, the morphology detector 236 may be utilized to identify non-conducted ventricular events, such as ventricular fibrillation and the like.

The arrhythmia detector 234 is configured to apply one or more arrhythmia detection algorithms for detecting arrhythmia conditions. By way of example, the arrhythmia detector 234 may apply various VF detection algorithms. The arrhythmia detector 234 is configured to declare a ventricular fibrillation (VF) episode based on the cardiac events.

The therapy controller 233 is configured to perform the operations described herein. The therapy controller 233 is configured to identify a multi-phase VF therapy based on the ventricular fibrillation episode, the multi-phase VF therapy including shocks and a pacing therapy. The therapy controller 233 is configured to manage delivery of the burst pacing therapy at a pacing site in a coordinated manner after the shocks. The pacing site is located at one of a left ventricular (LV) site or a right ventricular (RV) site.

The therapy controller 233 is further configured to analyze a timing of VF beats to obtain at least one of a VF cycle length (CL) or variation and to determine at least one of a number of pulses in a pulse train of the burst pacing therapy or a duration of pulse train of the burst pacing therapy based on at least one of the VF cycle length or variation. The therapy controller 233 may be further configured to set a timing delay to time the burst pacing therapy such that one or more of pulses therefrom occur during a period of time in which a local tissue region surrounding the pacing site is excitable and not refractory. The therapy controller 233 may be configured to set a frequency of the burst pacing therapy at a high frequency relative to a cycle length of non-fibrillation arrhythmias.

In accordance with embodiments, the S-IMD 100 may represent a subcutaneous implantable cardioverter defibrillator (S-ICD). Optionally, the communication modem 240 may be configured to wirelessly communicate with a leadless pacemaker, such as to pass timing information there between. The S-ICD may deliver phase I and II therapies, while the phase III pacing therapy may be delivered by the S-CID or the leadless pacemaker. The communication modem 240 may transmit timing information to a leadless pacemaker such as when sending an instruction for the leadless pacemaker to deliver pacing therapies in connection with embodiments herein.

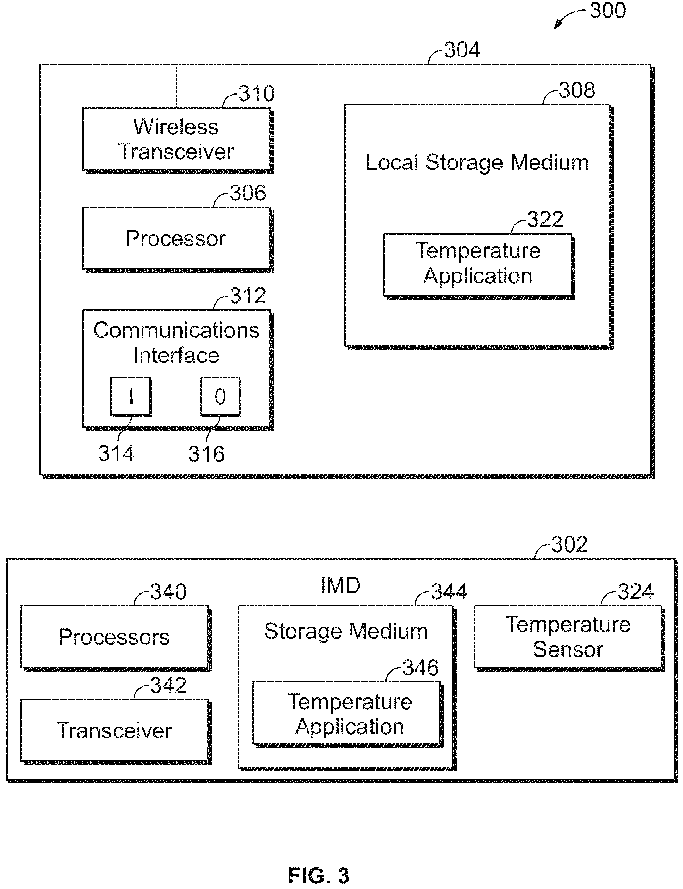

Figure 3 illustrates a control system 300 for an IMD 302. In one example the IMD 302 is the S-IMD of Figures 1-2. The control system 300 includes remote control 304, that in one example is the external device of the S-IMD of Figures 1-2 and illustrated in Figure 2.

The remote control 304 includes components such as one or more processors 306 (e.g., a microprocessor, microcomputer, application-specific integrated circuit, etc.), one or more local storage medium (also referred to as a memory portion) 308, one or more wireless transceivers 310, a user interface 312 which includes one or more input devices 314 and one or more output devices 316. All of these components can be operatively coupled to one another, and can be in communication with one another, by way of one or more internal communication links, such as an internal bus.

The local storage medium 308 can encompass one or more memory devices of any of a variety of forms (e.g., read only memory, random access memory, static random-access memory, dynamic random-access memory, etc.) and can be used by the processor 306 to store and retrieve data, including temperature data. The data that is stored by the local storage medium 308 can include, but need not be limited to, temperature sensor data, physiological sensor data including biometric data, diagnosis data, trend data, treatment data, therapy data, patient data including patient medical history data, or the like. Temperature sensor data can include temperature readings, changes in temperatures, changes in temperature over time, additional temperature data or the like. Temperature data can include average temperature, maximum and minimum temperatures, temperature changes, rate of temperature changes, etc. Physiological sensor data can include ECG or other cardiac activity signal data such as QRS length, peak data, frequency data, or the like. Physiological sensor data can also include accelerometer data related to patient position, activity, etc. The patient data can include diagnosis, age, medical conditionals, medications, or the like. Each operating system includes executable code that controls basic functions of the remote control, such as interaction among the various components, communication with the IMD 302 via the wireless transceivers 310, and storage and retrieval of applications and data to and from the local storage medium 308.

The input and output devices 314, 316 may each include a variety of visual, audio, and/or mechanical devices. For example, the input devices 314 can include a visual input device such as an optical sensor or camera sensor, an audio input device such as a microphone, and a mechanical input device such as a keyboard, keypad, selection hard and/or soft buttons, switch, touchpad, touch screen, icons on a touch screen, a touch sensitive areas on a touch sensitive screen and/or any combination thereof. Similarly, the output devices 316 can include a visual output device such as a liquid crystal display screen, one or more light emitting diode indicators, a mechanical output device such as a vibrating mechanism, etc. The display may be touch sensitive to various types of touch and gestures. As further examples, the output device(s) 316 may include a touch sensitive screen, a non-touch sensitive screen, a text-only display, a smart phone display, and/or any combination thereof.

The local storage medium 308 in one embodiment stores various content including, but not limited to a temperature application 322. The temperature application 322 includes executable code that utilizes operating systems to perform numerous functions related to obtaining and analyzing temperature data from a temperature sensor 324 of the IMD 302. In one example the temperature data obtained is treatment temperature data that is utilized to vary or change the treatment delivered by the IMD. Such treatment changes can include changes in treatment profiles, pacing profiles, IMD settings, or the like. In another example the temperature data can be diagnostic temperature data that can be used along with other information, diagnosis data, or the like to make diagnoses of sickness, illness, heart failure, heart failure risk, stroke, stroke risk, arrythmia, disease, etc. The executable code can be configured to analyze temperature data and make determinations related to diagnoses, treatments, or the like.

In one example, the user interface 312 can enable and disable the temperature monitoring function by implementing different operating modes of the IMD. Obtaining temperature data from the temperature sensor takes energy from the battery, or power source of the IMD 302. As a result, the temperature sensor 324 may not continuously operate. The temperature application 322 can include an "ON", or treatment mode where treatment temperature data is obtained and analyzed to make treatment changes of the IMD 302. In the treatment mode the system 300 continuously and/or periodically monitors and obtains temperature reading of the body, and the pacing rate is set, changed, varied, etc. based on the obtained temperature readings. In one example, the temperature reading (e.g., temperature data) can be used along with other variables to determine changes in the pacing rate. For example, the IMD can adjust pacing for a fever caused by an infection by using both temperature data that includes daily temperature maximums, daily temperature minimums, daily temperature median points, daily temperature average, daily circadian temperature cycle data for a temperature change profile, along with QRS characteristics. When the patient temperature reaches a threshold temperature, the IMD can use the QRS characteristics to adjust the VV delay narrowing the QRS complex to account for the infection induced fever.

In another example, when suspecting high fever associated with certain disease such as covid, flu, or the like, previous activity level and posture measurements can be utilized when providing additional pacing support (increase rate for example).

In yet another example, temperature data can be used to verify physiological data. An activity sensor, such as an accelerometer, alone can have false positive response when traveling in a car on a bumpy road, or tapping on the pacemaker, etc. The temperature data obtained from the temperature sensor can be used to complement the activity sensor and reduce the false positive responses. In another example, the temperature data from the temperature sensor may be used along with another physiological sensor, such as a ventilation level sensor to reduce false positive responses.

In another example, when a fluctuation in temperature readings occurs and no other monitored variables change, an accompanying variation or change in the pacing rate can be provided. Alternatively, if no body temperature change is indicated, and no other monitored variables change the pacing rate is not altered. In one example, the additional variables can include activity level that can be determined utilizing an accelerometer, heart rate monitor, or the like.

In the treatment mode, the temperature application 322 can have treatment profiles or setting changes that can be automatically implemented based on changes in the body temperature of the patient. The profiles can include rate, rise time, duration, and recover time for implementing the treatment based on the temperature data. In one example the profile can be determined prior to the change in temperature being obtained. Such treatment profiles can thus be obtained using the temperature change, body temperature, temperature threshold, etc. and utilizing a lookup table, decision tree, or the like. Alternatively, a determination can be made using the temperature data to determine the profile, or changes in profile. The determination can be made using a mathematical model, algorithm, artificial intelligence algorithm, mathematical function, or the like. The determination can be based on the temperature data by itself or based on temperature data along with other biometric data. Other biometric data can include patient age, known medical conditions, heart rate, oxygen levels, respiration rate, blood pressure, known medications, or the like.

In another example, the temperature application 322 can include a passive, or diagnostic mode where diagnostic temperature data is periodically obtained so that it can be used with other information, patient data, sensor data, etc. to make a diagnosis. In another example, the diagnostic temperature data may be obtained at a predefined time. The analysis and diagnosis can be made by the one or more processors 306, by a clinician reviewing the diagnostic temperature data, or the like. In a diagnostic mode the system 300 keeps a log to track the body temperature change (e.g., a long-term trend and circadian variation trend, an average, a minimum and maximum during a time window, or the like) as well as the corresponding intrinsic and pacing rate. The trending data can then be combined with, compared to, analyzed in relation to, etc. 1) exercise data trending, for example, to provide diagnostics for fever related activity decrease and heart rate increase; 2) pulmonary edema monitoring including impedance trending, for example, to provide diagnostics pneumonia related pulmonary edema excursion; 3) heart condition burden trends including atrial (AT), atrial fibrillation, or the like; 4) pacing threshold trending; 5) sense amplitude trending; 6) rate trending; 7) or the like.

In yet another example, the temperature application 322 can include an off mode that can be utilized to prevent bad or inaccurate temperature data from being obtained. Such bad or inaccurate temperature data may be the result of a medical procedure such as an MRI scan being undertaken, or other reason that can result in faulty temperature data being obtained. Other such conditions that cause a patient to place the temperature application 322 in the off mode can include going into a sauna, exercising, sleeping, etc.

In one example, the temperature sensor 324 obtains temperature data periodically during portions of a day. For example, the temperature data may be collected from 10:00 PM to 5:00 AM every day because this is when an individual is sleeping. Alternatively, temperature data could be collected from 6:00 PM to 7:00 PM or times when the patient is not sleeping. In yet another example, five minutes of temperature data could be obtained each hour. In other examples, a clinician may utilize the user interface 312 to begin obtaining temperature data as a result of a condition of a patient, a posture or activity change, a heart rhythm condition change, or the like. In one example, the patient may be sick, may have just had a procedure conducted that makes them more likely to have an infection, or the like such that temperature changes of the patient may be likely. In such instances the clinician may use the user interface 312 to put the IMD 302 and hence the temperature application 322 in a treatment mode.

In yet another example, the data and information obtained from other physiological sensors of the IMD can be utilized to have the remote control 304 to place the IMD in a treatment mode and begin receiving temperature data. For example, if an accelerometer detects an instance of a fall or syncope, if a heart rate monitor indicates a speeding or slowing of a heart rate, or the like, these are all indications that a fever, health condition, etc. is occurring where the body temperature of the patient may fluctuate, and a treatment may need to be altered. The determination, or receiving of data or information, cause the remote control 304 to automatically begin obtaining temperature data.

The temperature application 322 can be configured to vary a pacing treatment delivered by the IMD 302 to mimic the natural response of a healthy heart to human body temperature change, as well as providing monitoring functions. When in a treatment mode, the temperature application 322 can increase/decrease/regulate the pacing rate based on the body temperature to mimic the natural response of a healthy heart. The temperature application 322 can use trending diagnostics related to body temperature and body temperature changes as an additional factor that can affect the heart conditions or reflect a disease state. In this manner in a diagnostic mode, additional diagnostic capabilities are provided.

While in example embodiments the remote control 304 includes the temperature application 322, in other example embodiments the IMD 302 includes one or more processors 340, a transceiver, 342, and a storage medium 344 that includes a temperature application 346. The temperature application 346 of the IMD can be configured to provide the same functionality as described in relation to the temperature application 322 of the remote control 304. To this end, in example embodiments both the remote control 304 and the IMD 302 can each have a temperature application 322 that communicate with one another to perform the functionality and processes previously described. Alternatively, only one of the IMD 302 or remote control 304 have a temperature application 322 or 346 and perform the functionality and processes described.

For example, the IMD can be a dual chamber ICD programmed for dual chamber pacing with an initial AV delay of 200ms for primary prevention and intermittent conditional heart block. In a treatment mode that temperature application can obtain temperature data from the temperature sensor 324 along with physiological data from physiological sensors, physiological data communicated to the IMD, stored in the storage medium 344, or the like. The temperature application can then be configured to have a threshold temperature, such as 39 C°. Upon reaching the threshold temperature, the temperature application can be configured to alert a clinician by providing a communication via the transceiver 342 to the remote control 304, a remote device, etc. By providing alerts, a clinician can monitor the number of alerts along with physiological data, such as activity data, impedance data, heart rate data, or the like. The temperature application 346 can use the temperature data and physiological data to diagnose degeneration of lung function, heart block, pneumonia, or the like. Once diagnosed, treatment of the ICD may be varied to treat the diagnosis.

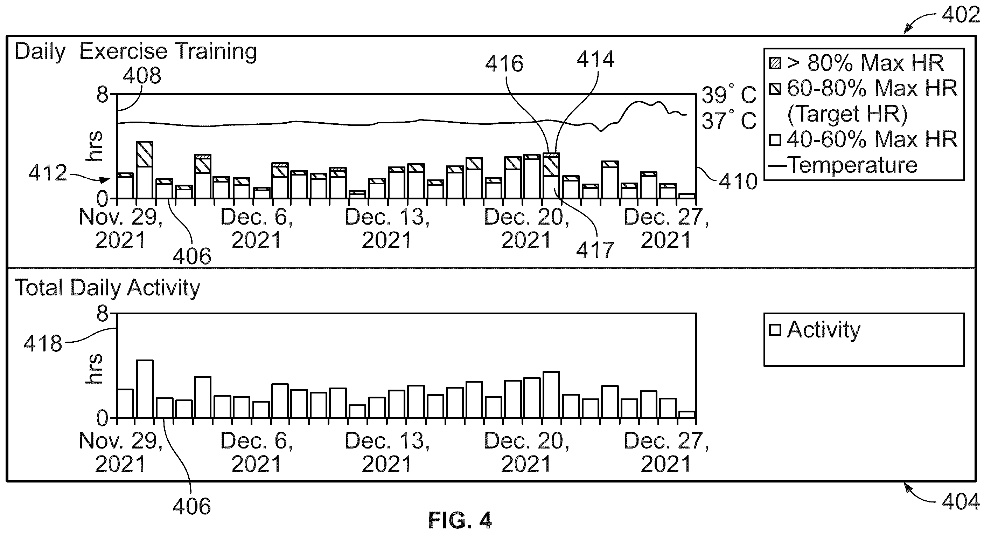

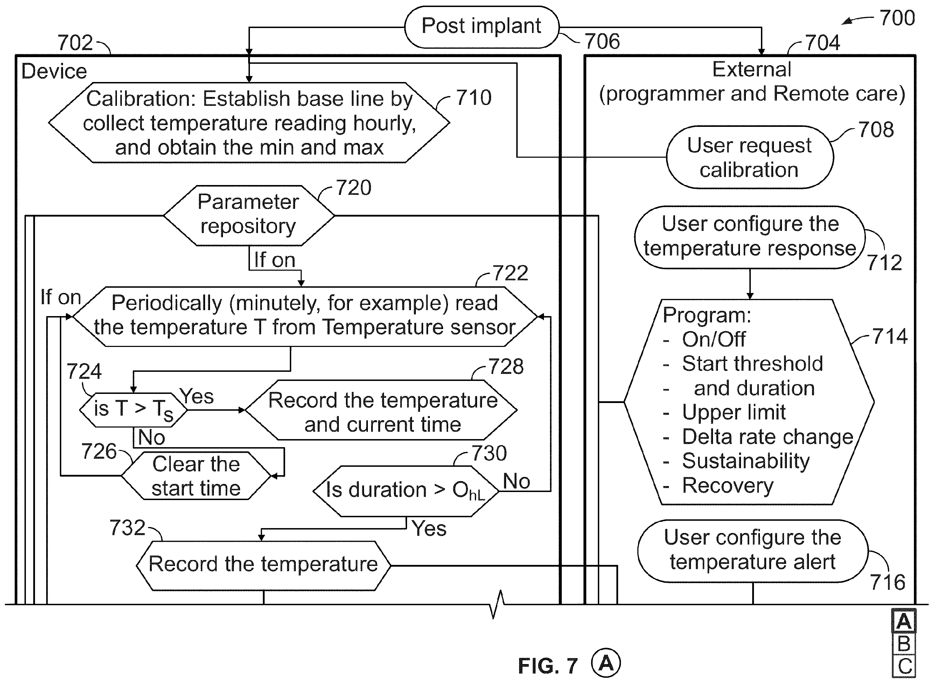

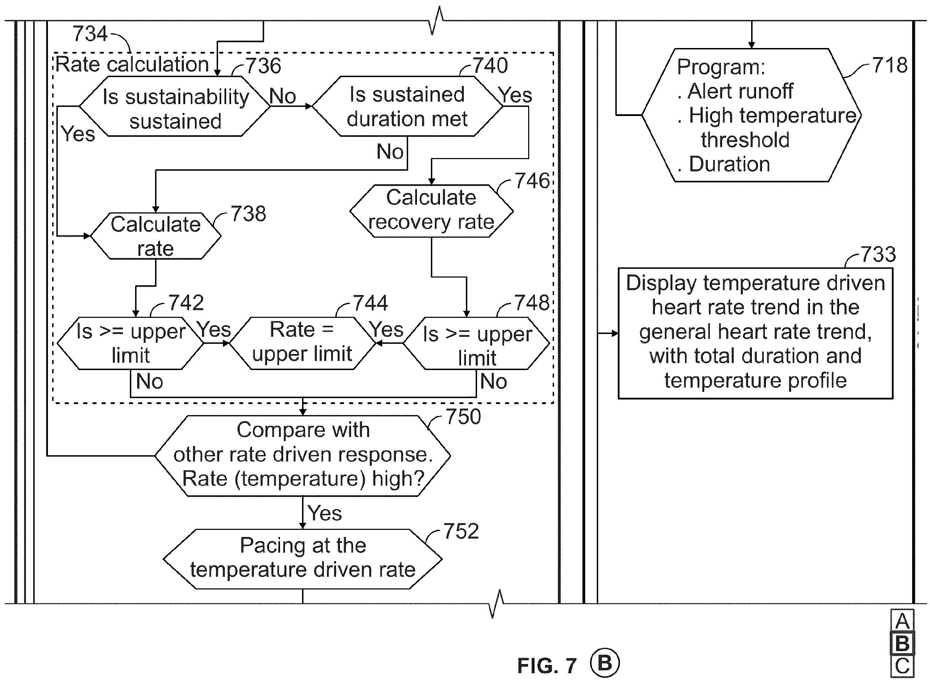

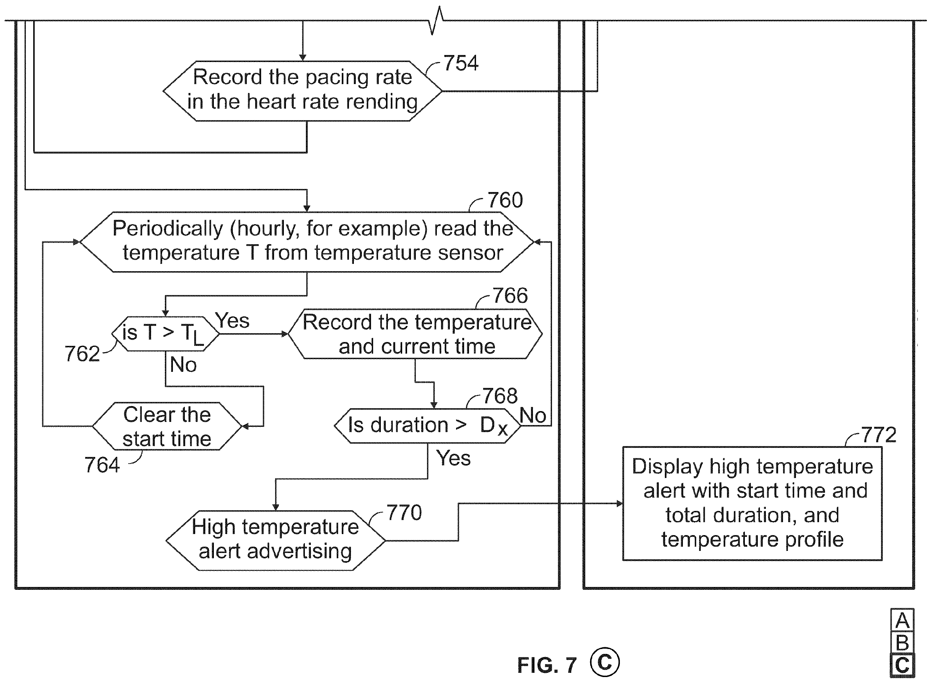

Figures 4-6 illustrate different graphs related to temperature readings of a temperature sensor that show how patient temperature fluctuations, or changes can be utilized to diagnosis physiological conditions of the patient when a diagnostic mode is utilized. Figures 4-6 illustrate different trends that a temperature application may use in combination with temperature data to make determinations related to treatments such as changing pacing for a patient. In one example, the temperature sensor that obtains the temperature reading provided in the graphs of Figures 4-6 is a physiological sensor of the S-IMD of Figures 1 and 2 and the control system of Figure 3. By using the temperature sensor to diagnose physiological conditions of the patient, treatment can be varied and updated accordingly (as is described in greater detail in Figure 7).

Figure 4 illustrates a top graph 402 related to the daily exercise training of a patient and a lower graph 404 that is related to the total daily activity of the same patient during the same time period. The top graph 402 shows an X-axis 406 related to time that is measured in increments of weeks with the first Y-axis 408 also measuring time, but this is the number of hours of daily exercise for the patient. The hours of daily exercise may be logged by the patient, determined based on heart monitoring signals that indicate an increase in heart rate, a combination of physiological data, or the like. The second Y-axis 410 meanwhile illustrates the body temperature of the patient measured in degrees centigrade (C°). In one example the body temperatures may be taken by any of the systems or devices described in relation to Figures 1-3. Each individual bar 412 of the top graph 402 represents one day for the patient. Each bar 412 is broken up to show the type of exercise that is being undertaken by the patient. In this example, daily exercise is being considered as activity, where the patient has a heart rate that is at least 40% of the maximum heart rate of the patient during that period. If the maximum heart rate during a period is 150 beats per minute (bpm), and a heart rate above 40% of that maximum is considered an active heart rate. Each bar 412 has a most active section 414 that is within 80% of the maximum heartbeat, a next most active section 416 are heartbeats at a rate between 60% and 80% of the maximum heartbeat, and a final section 417 that is between 40%-60% of the maximum heartbeat. Different exercises are captured, including walking, running, jogging, or the like.

The bottom graph 404 meanwhile has the same X-axis 406 of time measured in increments of weeks. The bottom graph 404 only has a Y-axis 418 that measures the total activity in hours. The activity in one example is measured in the same manner at the top graph and is related to the bpm of the heart, while alternatively, another manner such as measured body position changes obtained from an accelerometer, or other manner can be utilized to obtain the activity level of the patient.

As both the graphs 402, 404 of Figure 4 illustrated, during a period when the patient has an increased/fluctuating temperature, a correlation exists between the change in temperature and the reduction in activity of the patient. The correlation can be used to determine that the patient is experiencing a fever, and whether the pacing treatment needs to be altered or varied. If the IMD is in a treatment mode and makes this determination, treatment profiles can be selected based on the correlation between the increases in temperature and lack of activity to represent a period of time where a fever is occurring.

In another example, activity level and higher body temperatures can be indicative of extreme heat exposure. By detecting a higher body temperature while in a diagnostic mode, low activity sensor reading, along with lower blood pressure, an alert, and/or treatment for heat exhaustion or stroke can be provided. In this manner, correlating the body temperature changes with other physiological data can result in a diagnosis and treatment of a serious condition in a faster manner than if temperature data is not utilized.

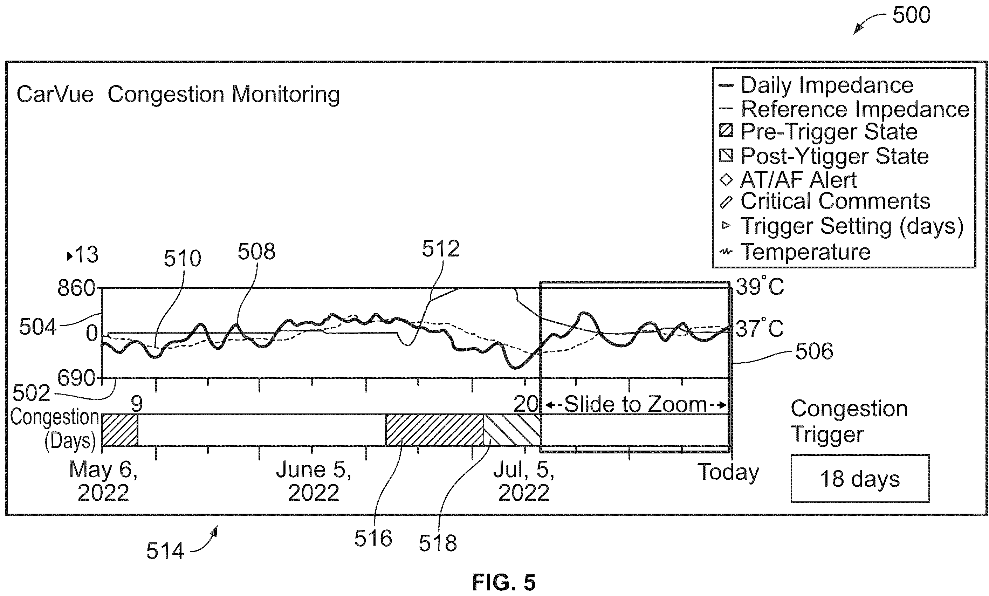

The graph 500 of Figure 5 illustrates yet another temperature correlation and how the system and devices of Figures 1-3 may be utilized to diagnose and treat health conditions of a patient. The graph 500 includes an X-axis 502 that measures time in months whereas the first Y-axis 504 measures pulmonary impedance measured in mmHg and the second Y-axis 506 measures temperature in C°. The graph 500 measures a daily impedance 508, reference impedance 510 along with the temperature 512. The graph also includes a lower bar 514 showing the same period of time and a pre-trigger state 516 along with a post trigger state 518. In this example, the association between the decrease of patient pulmonary impedance with the combined body temperature increase to a fever level indicates potential lung disease. The system can provide an alert to a clinician to test for or provide treatment for lung disease.

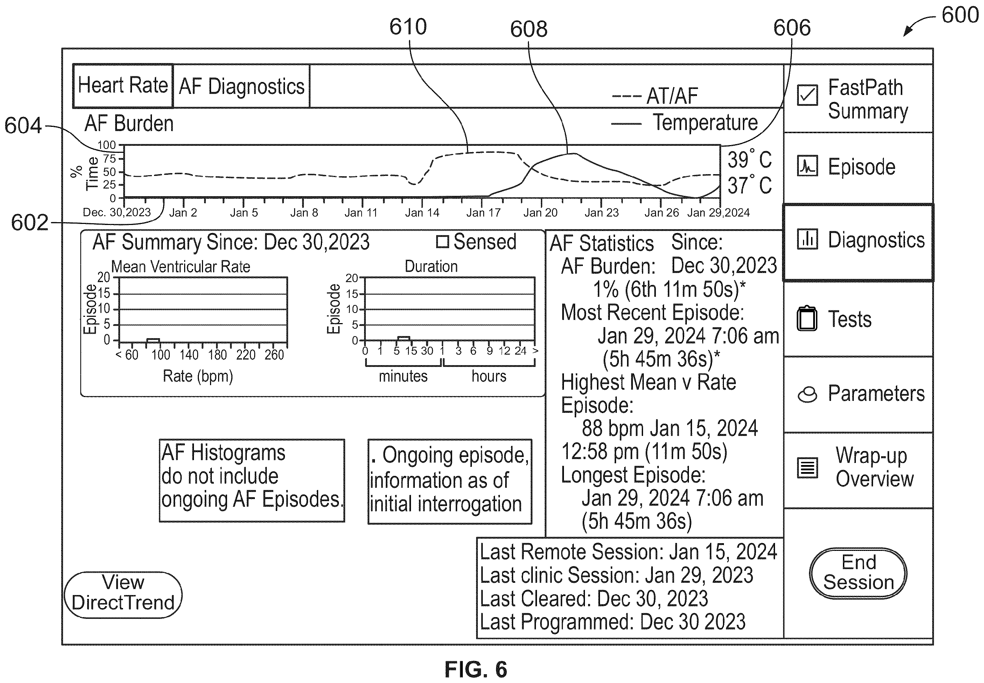

Figure 6 illustrates yet another example of a graph 600 where correlation between body temperature fluctuations and another physiological parameter can be utilized to diagnose and treat a patient. In this example the X-axis 602 again measures time. In this example, the time increments are provided in days.

The first Y axis 604 presents the percentage of time for each day the patient experiences an atrial fibrillation (AF) burden. An AF burden can be considered the total daily atrial tachyarrhythmia (AF, atrial flutter, atrial tachycardia, etc.) during in which the atrial rate exceeds a determined rate (beats per second, beats per minute, etc.). In other examples, the AF burden may be determined in other manners using other thresholds or considerations. The second Y-axis 606 presents body temperature and is measured in C°. Thus, a first measure 608 is provided that shows the patient body temperature over time while the second measure 610 shows the percent AF burden over time.

In this example, an association is illustrated between the increase of percent AF burden following a period of increased body temperature, or fever. The condition can indicate a latent or long term after effect of a disease associated fever. Fever sometime accompanies acute myocardial infarction and may be a pointer to the extent of infarction and the prognosis. Endocarditis is an infections cardiologic disease that is typically associated with such a fever. In this manner the system can provide an alert and/or treatment associated with endocarditis. When an IMD is in a diagnostic mode and obtains the diagnostic temperature data presented in the graph, an alert can be provided that allows a clinician to provide tests for endocarditis to then adjust or provide treatment accordingly.

The graphs of Figures 4-6 only show a few examples of how body temperature data can be utilized with other physiological data to make determinations related to diagnosing health conditions and treating a patient accordingly. Numerous other examples are additionally provided. Additional examples include a lower body temperature can be used with lead impedance measurements and blood pressure measurements to detect worsening congestive heart failure condition. The worsening congestive heart failure may be an after effect of an infection disease's effect on cardiac tissue. In yet another example, body temperature data itself can be utilized to provide an alert for a patient. For example, if the body temperature reaches a threshold level, or stays above a threshold level for a determined period of time, such as five days, an alert can be provided to a clinician of the condition of the patient. If the patient is not already in a hospital or clinicians office, such patient may be contacted to have them admitted for observation and tests. The body temperature data can be utilized for numerous different diagnostic and treatment purposes that improves current methodologies.

### Control System Algorithm and Functionality

Figures 7A-B provide a block diagram of the operation 700 of an IMD 702 that includes a body temperature sensor and an associated remote control 704 that can provide commands, settings, operations, etc. for the IMD 702 related to the body temperature sensor. In example embodiments, the systems, devices, IMDs, remote controls, or the like of Figures 1-3 perform one or more steps of the operations described in the operation 700. While not illustrated in the operation 700, each of the IMD 702 and remote control 704 includes processors, storage devices, transceivers, interfaces, temperature sensors, temperature applications, circuitry, inputs, outputs, etc. that are configured to perform the steps of the operation 700. In one example, the temperature sensor can be located at or on any component of the IMD or remotely coupled to the IMD. In another example, the IMD can have a lead upon which the temperature sensor is located or can be a leadless IMD and not located on a lead.

At 706, the IMD 702 is implanted into a patient. The IMD can be any of the IMDs previously described herein, including an ICD, ICM, or the like. Post implant the remote control 704 and IMD 702 can be paired with one another. In example embodiments, the IMD or remote control can be taken out of a shipped setting, a lead impedance can present a certain range, or the like. Such pairing can include the establishment of secure communication pathways, including using key exchange, cryptography, passcodes or passwords, etc. Such pairing and accompanying processes are not the subject matter of the current disclosure.

At 708, after a communication pathway is provided post implant, one or more processors of the remote control 704 can be utilized by a user to request calibration of the body temperature of the patient. Once the IMD is implanted, baseline temperature readings of the patient are requested to be taken. Not every patient has the same core body temperature. Initial readings need to be obtained or collected to improve the effectiveness of the process. For example, once the pairing occurs the IMD can be taken out of a shipped setting a begin periodically obtaining or collecting body temperature data of the patient. In one example, a reading can be obtained once every hour, once every two hours, once every six hours, once every twelve hours, once a day, or the like. An average can then be provided over a determined period of time such as one week, two weeks, one month, etc. to provide the baseline body temperature for the patient.

At 710, the one or more processors of the IMD obtain the baseline temperature data from the temperature sensor. In one example, the temperature data is obtained in response to a command or request by the remote control 704 at 708. In another example, the one or more processors of the IMD automatically begin obtaining baseline temperature data. In one example, after a determined amount of time, such as one week after implant, the IMD can automatically begin obtaining the baseline temperature data even without a command from the remote control 704. To obtain the baseline temperature data, the IMD can inquiry, or obtain baseline temperature data once every hour, once every day, ten times a day, or the like. A determined number of readings may be obtained, such as twenty, thirty, fifty, etc. to ensure accuracy of the baseline temperature data. Minimum and maximum readings can be obtained and utilized to determine temperature thresholds to be utilized during this process.

At 712, the one or more processors of the remote control 704 are commanded by a user to provide how the IMD 702 should operate in response to changing or varying body temperature readings. The user can be a clinician, technician, or the like and provide the settings of a temperature application during use. Based on patient physiological parameters such as age, weight, medical conditions, heart condition, or the like, the response to different temperature changes can be determined. For example, for one patient a temperature change threshold to begin compensating for the temperature change can be 1°C, whereas for another patient the temperature change may be 1.5°C.

At 714, the different settings of the temperature application can be provided. The temperature application can include three different modes of operation - a treatment (or on) mode, a diagnostic (or passive) mode, and an off mode. In the first mode, or treatment mode, treatment temperature data is obtained and the IMD (e.g., pacemakers, ICDs, etc.) provides treatment for a patient in the form of mimicking a natural response of the patient based on an elevated body temperature. For example, the temperature application, or feature, when programmed in a treatment mode increases/recovers the heart rate of a patient based on the body temperature to mimic the natural response of a human to elevated body temperature. Meanwhile, when in a second mode, or a diagnostic mode, the system passively obtains diagnostic temperature data that can be used together with other health metrics, or physiological data such as lead impedance measurement, activity sensor data, blood pressure etc. for diagnostic purposes. If while in a diagnostic mode an elevated temperature is detected, communications, alerts, or the like can be provided to a clinician for treatment purposes.

A user such as a clinician, patient, physician, or the like can program the system and thus temperature application to set it treatment/diagnostic/off depending on the clinic scenario. The programming in one example can be provided via a user interface where an input button can be pressed or actuated to provide the setting. For example, a clinician, patient, etc. may provide the treatment mode with the temperature application to provide temperature modulated cardiac pacing when the patient potentially has a fever. On the other hand, a clinician may turn off the system when the patient goes through the MRI scanning to prevent obtaining temperature data that may cause inaccurate determinations. In another example, the system may include a sensor, detection device, etc. configured to automatically determine that the patient is in an MRI scan and in response turn off the system until the MRI scan is no longer detected. In another example, the temperature application may include an MRI setting related to the use of the temperature data during an MRI scan. When the system is set to diagnostic mode, the system can record the temperature / temperate change but not make additional change in rhythm management. In the diagnostic mode the diagnostic temperature data can be obtained for diagnostic purposes, such as those described in relations to the graphs of Figures 4-6 where the diagnostic temperature data is correlated with other physiological data to provide alerts, diagnosis, treatment, etc. for particular medical conditions. In one example, the diagnostic temperature data obtained while the system is in diagnostic mode can be utilized to automatically change the mode of the system from diagnostic mode to treatment mode in order to begin treatment for the patient.

When in the treatment mode, the one or more processors of the IMD can increase the frequency of treatment temperature data collection. For example, when establishing a baseline temperature, or in a diagnostic mode, the diagnostic temperature data may be obtained at a first rate such as once an hour, day, etc. Whereas when in the treatment mode, the treatment temperature data may be obtained at a second faster rate such as once a minute, once every five minutes, once every ten minutes, etc. Increased treatment temperature data collection can be provided when the IMD is in the treatment mode for temperature collection and analysis compared to the diagnostic mode.

Additionally, in the treatment mode, an upper rate limit for pacing treatment can be provided. For example, the upper rate limit can be set to provide a pacing rate due to body temperate changes to 100 paces per minute (ppm). Alternatively, the upper limit can vary, including 60 ppm, 180 ppm, or the like depending on the patient. For example, the upper rate limit may depend on the age of the patient such that the upper rate limit is different for an infant than a teenager, thirty-year-old, eighty-year-old, etc.

The system can include a threshold temperature, or start temperature, for beginning pacing treatment. When the treatment temperature data indicates the body temperature of the patient has reached the threshold temperature, a new pacing rate can be determined. The temperature threshold can include an upper threshold that indicates fever, or a lower threshold that indicates hypothermia. In each instance, once the threshold is reached, a new pacing profile (e.g., treatment) is utilized that is based on the treatment temperature data being obtained.

In another example, the system can include a change or delta rate change per temperature change. In this manner, if a temperature fluctuates above a range threshold, a new pacing profile may be determined. In particular, a setting can be provided of a delta change per degree of increase or decrease of a temperature. In one example the delta can be a linear function, provided in a lookup table, or the like.

In yet another example, the interface of the system can include a response time for determined temperature changes. A user setting can be provided to set response time, or how long before an alert is acknowledged, or a treatment is started. If a patient has an increase in temperature during a determined period, such as twenty-four hours, a first response time can be provided. Alternatively, if the same temperature change occurs, but the increase happens over three days, a second response time can be provided. To this end the user can determine the threshold temperatures, determined period of time, response time, etc.

In one embodiment, the interface of the system can also allow a user such as a clinician to provide a setting for a duration or determined period of time for an increased heart rate in response to the temperature change. In particular, such an increased heart rate while mimicking the natural heart rate of a healthy individual can have its own negative health effects. A setting is provided to set a determined period, such as twelve hours, one day, three days, or the like to continue to provide pacing that results in an increased heart rate. In one example, the system can have a maximum beats per minute for a setting, and once that maximum rate is reached, the system may decrease pacing to decrease the heart rate a determined amount. In one example, the heart rate may be decreased one beat per minute every hour after reaching the maximum rate until normal pacing is reached. Alternatively, the setting may allow the maximum beats per minute to continue for a determined period such as three hours, four hours, twelve hours, etc. before any decrease occurs. In other examples, the decrease that occurs can be greater or less than one beat per hour. A decrease of two, three, five, etc. beats per hour can occur. In this manner the system can be customized for the individual patient to balance different health risks and depending on the desired treatment desired by the clinician.

In yet another embodiment a user can provide a determined amount of time to recover back to the treatment profile being utilized before changes as a result of temperature changes. As indicated above, a user can decide to decrease the beats per minute for treatment of the elevated temperature. In addition, if body temperature decreases, and the patient is showing recovery, a setting is provided for increasing the rate at which the system provides the treatment profile that was provided before changes were made because of the temperature increase. As body temperature returns to normal, the speed at which the recovery to the initial pacing profile can similarly be speed up to reflect the change of the body temperature.

In addition to temperature response configurations, a user at 716 can provide the one or more processors of the remote-control alert settings. As illustrated at 718, the one or more processors of the remote control obtains settings such as whether an alert should be provided based on a sustained temperature reading being greater than a threshold temperature longer than a threshold duration. In this manner the user can determine the threshold temperature, and the duration before the alert is provided. The setting can vary based on physiological parameters related to the patient. For an older patient in poor health, such alert threshold temperature may be 1°C with an alert threshold duration of 6 hours, whereas for a younger healthier patient the alert threshold temperature may be 1.5°C and the alert threshold duration may be two days.

Once all of the temperature response settings are provided by the user at 714 and 718, at 720 the one or more processors of the IMD store these settings, or parameters in a repository, or storage device. A clinician can use the remote control to provide determined settings for a patient that best meet the needs of that patient.

Once the settings are provided a 720, if the temperature application is in a treatment (or active) mode, then at 722, the one or more processors periodically receive treatment temperature data from the temperature sensor. In one example, treatment temperature data is obtained from the temperature sensor once every minute. In another example the treatment temperature data can be obtained once every 5 minutes, 10 minutes, 15 minutes, etc. In examples the periodic time increment is less than once every hour, less than once every 30 minutes, less than once every 10 minutes, less than once every 5 minutes, etc. Still, in the treatment mode the one or more processors obtain the treatment temperature data frequently, and more frequently than when the temperature application is in a diagnostic mode. In the treatment mode the temperature application is actively adjusting and varying treatments, such as pacing of the patient based on a change in body temperature from the baseline body temperature. Still, once the body temperature reverts back to the baseline body temperature, the changes in treatment are no longer needed, and the IMD changes back to the original settings. To this end, providing varied treatments based on temperature, such as increased beats per minute may actually be harmful to the patient if provided when the body temperature of the patient is no longer elevated (or decreased). As a result, the treatment temperature data from the sensors must be obtained frequently to ensure as soon as a fever breaks, a body warms after hypothermia, etc. the treatment returns to normal.

At 724, the one or more processors determine whether the most recent body temperature obtained exceeds a threshold temperature, along with a current start time. For example, if the baseline temperature of a patient is 37°C, the threshold temperature (Tₛ) may be 38°C, 38.5°C, 39°C, 39.5°C, etc. In addition, the time of taking the temperature reading is also important because fluctuations in body temperatures can occur based on environmental conditions, hardware conditions, or the like, so if the treatment temperature data presents a change before any changes in treatment occur the change in temperature must last for a determined duration to verify the change in temperature is likely because of a physiological condition. If a determination is made that the threshold temperature has not been reached, then at 726 the start time is reset as the treatment temperature data continues to be periodically obtained at 722.

If at 724 the most recent body temperature does exceed the temperature threshold, then at 728 the temperature and time are recorded in a storage device. By recording the temperature data and time, this information can be analyzed with other treatment temperature data and time data to determine trends related to the data collected.

At 730, the one or more processors of the IMD determine whether a threshold duration has been reached. The one or more processors analyze the most recent temperature readings and the time each was recorded to determine whether a threshold duration has been reached. For example, the threshold duration may be ten minutes, and the processor may obtain a treatment temperature data once every minute. If ten straight readings of treatment temperature data indicate the threshold temperature has been exceeded, then the ten-minute threshold duration may be met. In another example, by having at least a determined number of readings, such as 8 readings that exceed the temperature threshold in a ten-minute span can result in the duration threshold being met. If the duration threshold is not met, then no additional action is taken, and treatment temperature readings continue to be obtained and analyzed. If at 730, the threshold duration is reached, then at 732 the one or more processors record the most recently obtained temperature.

In one example, at 733 the one or more processors of the remote control are in communication with the IMD to obtain and display the recorded temperature and time. In this manner a clinician can review the temperature of a patient and make real-time determinations related to the temperature recorded.

Once the temperature (that exceeds the threshold and duration requirements) is recorded, a heart rate adjustment process 734 for determining the desired heart rate of the patient is then utilized. As indicated above, because a diseased heart can have problems increasing heart rate in response to a fever, the system varies treatment to provide a heart rate that mimics the heart rate of a healthy individual when they experience a fever, hypothermia, body temperature change, etc.

During the heart rate adjustment process 734, at 736 the one or more processors determine whether sustainability is sustained. Once a temperature exceeds the temperature threshold, the one or more processors determine a temperature-based pacing rate that is R1=(T-T(0))* delta rate increase where T(0) is the threshold. The system then ramps up the pacing rate based on the respond time: delta_R=(R1-current_rate)/reaction time until the rate reaches the R1. Once reaching the intended pacing, if the rate sustainability is considered to be "Sustain," then the rate stays the same. If the rate sustainability is at "decay," then the rate slowly decreases over the decrease rate after a programmed duration. For certain patients there is not a desire to keep the heart rate of a patient elevated for too long a period of time, so the sustainability determines whether to keep a heart rate at an elevated rate, or to start reducing a heart rate. A duration may be determined and programmed by a clinician that is a determined high-rate duration. The determined high-rate duration is the duration that the heart rate of the patient can remain elevated at a maximum heart rate. In examples, the high-rate duration can be one hour, two hours, one day, two days, or the like. Once the high-rate duration is reached without a reduction in the heart rate determined, the heart rate automatically begins reducing per a reduction setting. For example, the heart rate may be reduced by one beat per minute every minute until a normal pacing rate is reached.

Alternatively, if a temperature lowers, similarly, a new reduced rate can be calculated. To this end, these reduced rates are considered recovery rates. In one example, when the temperature decreases, a new temperature Tₙ is less than the previous temperature Tₚ, the system calculates the temperature pacing recovery rate as R2=(Tₙ (current) - Tₚ (previously)). As such a delta rate decrease is calculated per recovery time. If the temperature goes too high/low for a predefined duration D, (e.g., higher/lower than a predefined threshold such a 35.5°C or 42°C) an alert can be generated and sent via a remote care monitoring system to alert a clinician.

If at 736, the heart rate is to be sustained, then at 738 the heart rate is calculated based at least in part on the body temperature that has been obtained. Alternatively, if the heart rate is not to be sustained, then at 740 a determination is made whether a high-rate duration has been reached. As indicated above, if a patient is determined to be of such health that their heart should only be allowed to reach a maximum rate for a determined period of time, that determined period of time is the high-rate duration. If the high-rate duration has not yet been reached, then again at 738 the one or more processors calculate the heart rate.

Once the heart rate is calculated at 738, at 742 a determination is made whether the calculated heart rate is greater than a maximum heart rate (e.g., upper limit). The maximum heart rate, or upper limit, can be determined based on the physiological conditions of the patient to prevent the heart rate from becoming dangerously too high for the patient. If the calculated heart rate is equal to or greater than the upper limit, or maximum heart rate, the IMD only functions to pace the heart to reach that maximum heart rate, and not a greater rate. Consequently, at 744 the heart rate is set as the upper limit or maximum heart rate.

If at 740 a determination is made that the high-rate duration has been reached, then at 746 a recovery heart rate is calculated. The recovery heart rate provides reductions to heart rate based on the health conditions of the patient. Still, the calculation can still result in a calculated heart rate that is above the maximum heart rate. At 748, a determination is made whether the calculated recovery heart rate at 746 is greater than or equal to the upper limit, or maximum heart rate. If so, at 744, the heart rate is set as the upper limit or maximum heart rate.

During the heart rate adjustment process 734, a heart rate (whether set at the upper limit, or under the upper limit) is provided. Next at 750 the heart rate is calculated and compared to other rate driven responses for treatment to determine if the calculated heart rate is higher than the other rate driven responses. If not, the higher heart rate as determined by the other rate drive response is utilized, and the system continues obtaining treatment temperature data. If the calculated heart rate is greater than other calculated heart rates based on other variables, the calculated heart rate is selected as the heart rate.

If the calculated heart rate is selected as the heart rate at 750, then at 752 the one or more processors of the IMD provide pacing (e.g., treatment) at the calculated heart rate that is based on body temperature. Then, at 754, the one or more processors of the IMD record the pacing rate. In addition, at 733, an interface of the remote control can display on and output the patient body temperature previously recorded, along with the heart rate recorded at 754 to provide information to the clinician regarding the response to the body temperature fluctuation.

In an example, when the IMD operates in the treatment mode, temperate data may be continuously obtained by the IMD while the IMD provides an initial treatment. The collected temperature data may be an overall circadian temperature cycle that is compared to historical data within the memory of storage device of the IMD. The one or more processors of the IMD can determine based upon the temperature data (e.g., trending data), that the patient has an onset of atrial fibrillation associated with a body temperature increase to a determine threshold temperature, such as just under 39 C°. The one or more processors can determine that an atrial fibrillation burden increases over time and that there are also other cardiac conditions, such as VT 1 occurrences, detected by the IMD. Pacing treatment can be varied based on the temperature data to address the atrial fibrillation burden. Similarly, in other examples, the temperature data can be used to diagnose and treat polymorphic VT and VF, including of a patient with diabetes, along with other similar diagnoses and treatments.

In an example, the system also can function in the diagnostic mode that is also illustrated in Figure 7. In the diagnostic mode the system obtains and collects diagnostic temperature data and the diagnostic temperature data is obtained less frequently than the treatment temperature data. While in the diagnostic mode the system does not function to adjust or vary treatments/pacing based on changes in the body temperature of the patient. Instead, in the diagnostic mode temperature data is periodically collected to either use with other physiological information and data to facilitate diagnosis of health conditions, or to provide an alert of a potential health condition as a result of a detected changed body temperature.

In the diagnostic mode, once the settings have been provided and recorded at 718 and 720 then at 760, the one or more processors of the IMD periodically obtain diagnostic temperature data (T) from the temperature sensor. In one example, the IMD may obtain diagnostic temperature data from a temperature reading once an hour. In another example the IMD may obtain the diagnostic temperature data once every two hours. In yet another example, the frequency of obtaining the diagnostic temperature data may dynamically vary in real-time. For example, the diagnostic temperature data may be obtained at an initial rate of once every three hours. Then if a temperature increase threshold (e.g., .5°C, 1°C, etc.) is reached, the rate at which the diagnostic temperature data is obtained can be once every hour instead of once every three hours. The rate could increase as more elevated temperature readings are obtained or decrease back to an original rate if temperatures obtained decrease back to below the temperature threshold.

By collecting diagnostic temperature data in the diagnostic mode, not only can increases in body temperature be detected, but the diagnostic temperature data obtained can be utilized for temperature-based heart rate diagnostics. For example, when collecting daily temperature variation as well as weekly and monthly trends, health conditions can be identified. The system can keep a log to track temperature changes as well as corresponding intrinsic and pacing rate with a reasonable time resolution, such as hourly. The trend can then be provided, or displayed to a clinician during an office visit, follow up, or the like for diagnostic and treatment purposes. Long-term trend and circadian variation trend can be determined or identified using the periodic (e.g., hourly) body temperature measurements and validated by other physiological data such as activity data, posture data, etc. Another physiological sensor such as an accelerometer can be used to collect physiological data for additional trend information and compared to the corresponding diagnostic temperature data. Using such physiological data can also further refine temperature-based diagnosis by discarding or shifting the time to the rest/sleep time temperature can be aligned. In yet another example, other physiological data can result in identifying trends, including heart contraction characteristic trends, heart rate trends, QRS characteristic trends (including amplitude, width, or the like), capture threshold trends, A and V tachycardiac arrythmia trends, lung impedance trends, respiratory frequency trends, or the like can be analyzed in association with the collected body temperature data for the purposes of diagnosis and treatment.

For each reading obtained, at 762 the one or more processors of the IMD make a determination whether the temperature T is greater than a baseline temperature. If not, at 764 the start time recorded for obtaining the temperature reading is cleared and additional diagnostic temperature data is obtained at 762. In particular, if the body temperature of the patient has not increased there is no reason to record the data for diagnostic purposes, so instead of wasting memory and associated battery power, the time and temperature reading can be discarded accordingly.

If at 762 the temperature T is greater than a baseline temperature, then at 766, the one or more processors of the IMD record the temperature and the time of the reading. In this manner, the one or more processors begin recording the temperature and time for diagnostic purposes for the clinician, so the clinician knows when the body temperature of the patient started to rise, and how fast the body temperature of the patient increased. For example, if a body temperature only increases slightly and remains slightly elevated, a different health condition is likely presented than if the temperature increases rapidly and significantly from one reading to a next reading.

At 768, the one or more processors of the IMD determine whether a threshold duration has been reached. Once the one or more processors determine that the body temperature of the patient is elevated, then a determination is made for how long the temperature has been elevated. A threshold duration is provided, such as 4 hours, 5 hours, 6 hours, etc. If an increased temperature was detected because of an activity of the patient, a misreading, or the like, if the temperature does not remain elevated for the threshold duration, and there is no need to provide an alert. This is because when the body temperature is below the threshold temperature, there is no indication of infection or sickness. Still, if the temperature remains elevated for the threshold duration, then an indication is provided that an infection, sickness, or the like is present in the patient. In one example, the threshold duration may be dynamic and based on the temperature recorded above the baseline temperature. If a temperature increases by 3.5°C or more, just one temperature reading may be sufficient to result in an alert. While if the detected temperature increase is consistently between .5°C-1°C the threshold duration may be 24 hours. If the threshold duration is not reached at 768, then diagnostic temperature data continues to be periodically obtained until the duration is reached, or the threshold temperature is no longer exceeded.

If at 768 the threshold duration is reached, then at 770, the one or more processors communicate a high temperature alert or advertising to the remote control. In this manner, once a body temperature of a patient is considered elevated for a long enough period of time, an alert is communicated to let the clinician know of a potential health condition of the patient. To this end, at 772, the one or more processors of the remote control display the alert. In one example, the alert can include an auditory sound, haptic response, be presented in a different font, be presented with a color, or the like to gain the attention of the clinician. In addition, the alert may include recorded information such as start time of increased temperature readings, duration of increased temperature readings, temperature profile, or the like.

The system can be used for diagnosing and treating disease based on the temperature data that is obtained. The system can co-exist with an activity sensor-based rate adaptive pacing as well. The temperature-modulated cardiac pacing can co-exist in harmony with the activity sensor-based rate adaptive pacing. When the patient has fever, the patient is more likely to be less active than usual, and the temperature-modulated cardiac pacing is the most effective heart rate regulation in order to help dissipate the body heat. When the patient is doing light activities, the core body temperature of the patient most likely will not increase significantly, and the activity sensor-based rate adaptive pacing is the most effective response to compensate for the chronotropic incompetence.

The temperature driven pacing rate modulation, in conjunction with the traditional activity sensor driven rate modulation, provides a more comprehensive cardiac rate control for patients with various physiological and pathophysiological conditions. When there is no or very low activity while a temperature sensor indicates a need to provide more cardiac support, the temperature sensor-based modulation can further support the patient need for cardiac output. The temperature drive rate can be further regulated by a posture parameter that when in standing up position, the rate increase can be larger than when the patient is bedridden.

Another advantage of the system is disease status change monitoring and alerting. With collected temperature trending and a circadian variation trend, when a patient has prolonged time of temperature excursion - exceeding the upper limit, for example 1-2°C increase from the typical long-term trend and daily variation, or below the low limit (e.g., 1-2°C decrease from the typical long-term trend and daily variation), with relative low activity, and posture indicating a long laydown position, the alert can be (per programming) sent to indicate a disease status change (for example longer term infection or progress in heart failure resulting less cardiac output). The long-term temperature trending and the circadian variation trend provides the effectiveness of pacing therapy especially in CRT or CSP (conduction system pacing to treat HF), where the pacing improved cardiac output will reflect the body recovery from low body temperature. When the temperature trend indicates high temperature with elevated heart rate, the Heart Rate Variability (HRV) may reduce and a second evaluation of the trigger episode resulting from HRV can be performed post process to reduce the false positive rate of the AF detection.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for conducting operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices and program products according to various example embodiments. These program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being conducted in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A system (300) for managing temperature-based diagnosis and treatment based on temperature data, the system (300) comprising:
an implantable medical device, IMD, (100, 302) comprising a temperature sensor (253, 324) configured to collect treatment temperature data at a first rate when the IMD (100, 302) operates in a first mode and collect diagnostic temperature data at a second rate when the IMD (100, 302) operates in a second mode;
wherein the first rate is greater than the second rate; and
one or more processors (306, 340) configured to:
analyze the treatment temperature data when the IMD (100, 302) is in the first mode; and
deliver a treatment of the IMD (100, 302) based on the treatment temperature data;
analyze the diagnostic temperature data when the IMD (100, 302) is in the second mode; and
communicate an alert based on the diagnostic temperature data.

2. The system of claim 1, further comprising a remote control (304) in communication with the IMD (100, 302), the remote control (302) including one or more processors (306) configured to receive the alert communicated by the IMD (100).

3. The system of claim 2, wherein the one or more processors (306) of the remote control (304) are further configured to display the alert on a display (316) along with the diagnostic temperature data.

4. The system of claim 2 or 3, wherein the remote control (304) includes settings related to the treatment of the IMD (100), the diagnostic temperature data, and the treatment temperature data.

5. The system of any one of claims 2 to 4, wherein the one or more processors (306) of the remote control (304) are configured to switch the IMD (100) from the first mode or second mode to a third mode where no temperature data is collected.

6. The system of any one of claims 1 to 5, further comprising:
a physiological sensor (270) configured to obtain physiological data:
wherein in the second mode the one or more processors (306, 340) are further configured to:
identify a trend related to at least one of the diagnostic temperature data and the physiological data;
identify a change in health status of a patient based on the trend; and
communicate the alert in response to identifying the change in the health status.

7. The system of claim 6, wherein the physiological sensor (270) is at least one of a monitoring sensor, an activity sensor, or an impedance sensor.

8. The system of claim 6 or 7, wherein the IMD (100) includes the physiological sensor (270).

9. The system of any one of claims 6 to 8, wherein the trend is at least one of a heart contraction characteristic trend, heart rate trend, QRS characteristic trend, capture threshold trend, lung impedance trend, tachycardiac arrythmia trend, or respiratory frequency trend.

10. The system of any one of claims 1 to 9, wherein the one or more processors (306, 340) are configured to analyze the diagnostic temperature data by determining whether a change in a temperature threshold has been exceeded, and the one or more processors (306, 340) are configured to communicate the alert based on the change in temperature threshold being exceeded.

11. The system of claim 10, wherein the one or more processors (306, 340) are configured to analyze the diagnostic temperature data by determining whether the temperature threshold has been exceeded for a duration threshold, and the one or more processors (306, 340) are configured to, in response to the duration threshold being exceeded, communicate the alert.

12. The system of any one of claims 1 to 11, wherein the one or more processors (306, 340) are configured to deliver the treatment of the IMD (100) in the first mode based on the treatment temperature data using a first treatment profile for a determined period of time, and after the determined period of time, deliver the treatment of the IMD (100) based on the treatment temperature data using a second treatment profile.

13. The system of any one of claims 1 to 12, wherein the one or more processors (306, 340) are configured to, in respond to communicating the alert, change the IMD (100, 302) from operating in the second mode to operating in the first mode to collect the temperature data at the first rate.

14. The system of any one of claims 1 to 13, wherein the IMD (100, 302) comprises the one or more processors (340).

15. The system of any one of claims 1 to 13, further comprising a remote control (304) in communication with the IMD (100, 302), wherein the remote control (302) comprises the one or more processors (306).
